# EUROPÄISCHE PATENTSCHRIFT

(11) **EP 1 929 939 B1**
(45) Veröffentlichungstag und Bekanntmachung des Hinweises auf die Patenterteilung: **31.12.2014**
(21) Anmeldenummer: 07018771.1
(22) Anmeldetag: 25.09.2007
(51) Int. Cl.: A61B 1/00

(54) **Verfahren und Anordnung zur mikroskopisch hochaufgelösten Abbildung in der Laser-Endoskopie**
Method and assembly for microscopic high-resolution reproduction laser endoscopy
Procédé et agencement destinés à la représentation microscopique haute résolution en endoscopie laser

(30) Priorität: 29.09.2006 DE 102006046925; 28.09.2006 DE 102006046554
(43) Veröffentlichungstag der Anmeldung: 11.06.2008
(73) Patentinhaber: JenLab GmbH, 66119 Saarbrücken (DE)
(72) Erfinder: König, Karsten, Prof., Dr., 66119 Saarbrücken (DE); Tchernook, Andrei, Dr., 09212 Limbach-Oberfrohna (DE)
(74) Vertreter: Freitag, Joachim

(56) Entgegenhaltungen:
- WO-A-03/059563
- WO-A-2006/004743
- WO-A-2006/045936
- WO-A-2007/022616
- DE-A1- 10 065 146
- DE-A1-102006 046 554
- JP-A- 2003 344 777
- US-A- 5 738 676
- US-A1- 2004 260 148
- US-A1- 2006 195 072
- US-A1- 2007 081 236
- US-B1- 6 369 928

## Beschreibung

Die Erfindung betrifft ein Verfahren und eine Anordnung zur Erzeugung hochauflösender mikroskopischer Abbildungen in der Endoskopie auf Basis von lasergenerierter Objektreaktionsstrahlung und zur Erzeugung mikroskopischer Bearbeitungsschritte.
Die Erfindung findet vorzugsweise Anwendung in der Laser-Endoskopie auf der Basis von Multiphotonen-Prozessen mittels Femtosekunden-Lasertechnik.
Sie ist insbesondere einsetzbar bei Mikroabbildungverfahren, Laser-Scanning-Mikroskopie, optisch-kohärenter Tomographie sowie Ein- und Multiphotonen-Imaging, und findet wegen ihrer weitgehenden Miniaturisierung und Flexibilisierung des abbildenden bzw. bearbeitenden optischen Systems insbesondere Anwendung in der Endomikroskopie und Endomikrochirurgie.
Die Erfindung eignet sich besonders für die präzise Bearbeitung von biologischen Materialien, wie die optische Desaktivierung unerwünschter Zellen in einem Zellverband, die Bearbeitung des Augenhintergrundes und der Augenlinse, die Bearbeitung von Implantaten, die kontrollierte Wirkstoffabgabe, die Kieferchirurgie, die HNO-Chirurgie, die Gefäßchirurgie, Lymphknotentherapie, Herzchirurgie, Neurochirurgie, Stammzelltherapie und Tumortherapie.

Strahlung von Femtosekundenlasern wird bislang vorwiegend im diagnostischen Bereich eingesetzt. Insbesondere wird die mittels naher infraroter (NIR) Femtosekundenlaserstrahlung induzierte Zweiphotonenfluoreszenz (siehe z.B. US 5,034,613 A) und SHG für eine dreidimensionale Mikroskopie biologischer Objekte genutzt. Zudem werden Femtosekundenlaser für die Diagnostik mittels optischer Kohärenztomographie (OCT) eingesetzt (WO 1998/038907).
NIR-Femtosekundenlaser für die optische Bearbeitung mit einer Präzision im Sub-Millimeterbereich werden bislang lediglich für die Cornea-Behandlung (z.B. EP 1 470 623 A2, DE 101 48 783 A1, US 5,993,438 A) kommerziell eingesetzt. Dabei werden Multiphotonen-Prozesse wirksam, die zu einer Ionisierung des Targets, einem optischen Durchbruch, einer Plasmabildung und zur Entstehung disruptiver Prozesse wie die Ausbildung und den Zerfall von Kavitätsblasen und die Generation von Schockwellen führen und für eine Materialbearbeitung genutzt werden können. Durch die beugungsbegrenzte Fokussierung der Laserstrahlung mit Fokussieroptiken einer hohen numerischen Apertur (NA > 1) auf Beleuchtungsspots unter einem Mikrometer Durchmesser reichen NIR-Laserimpulse geringer Nanojouie-Impulsenergie aus, um den Schwellwert für den optischen Durchbruch im typischen Bereich um 1 TW/cm², um Materialbearbeitungen vorzunehmen. Es wurde demonstriert, dass durch die Anwendung von multiplen -1 nJ Impulsen, Schneidwirkungen und Bohrungen im Sub-200 nm-Bereich ohne Kollateralschäden realisiert werden können (KÖNIG et al., Optics Express 10 (2002) 171-176, KÖNIG et al., Med. Laser Appl. 20 (2005) 169-184).
Zusätzlich zur medizinischen Anwendung im Bereich der Behandlung des vorderen Augenabschnittes existieren Femtosekunden-Laseranordnungen für die präzise Oberflächenbearbeitung von Halbleitern und anderen Werkstoffen (LeHarzic et al., Optics Express 13 (2005) 6651-6656).
In der DE 100 65 146 A1 wird ein Verfahren zur Analyse und optischen Bearbeitung von pigmentierten Hauttumoren mittels intensiver NIR-Femtosekunden-Laserstrahlung und Fokussieroptiken hoher numerischer Apertur (NA) beschrieben. Der Einsatz im Targetinneren ist auf den Arbeitsabstand der Makro-Fokussieroptik mit hoher numerischen Apertur 1,2 ≤ NA ≤ 1,3 von typischerweise 200 µm beschränkt (KÖNIG, RIEMANN, Journal Biomedical Optics 8 (3) (2003) 432-439).
Bislang existieren keine kommerziellen Lichtendoskope mit Fokussieroptiken hoher numerischer Apertur. Typische numerische Aperturen liegen im Bereich kleiner NA ≈ 0,3. Zudem basieren alle kommerziellen Licht-Endoskope auf optischen Materialien und Lichtleitern, die infolge hoher Dispersion keine Übertragung von Femtosekunden-Impulsen ermöglichen.
Es existieren erste Endoskop-Prototypen für den Einsatz an Kleintieren auf der Basis von GRIN-Linsen und mikrostrukturierten Lichtleitern geringer NA für eine Zweiphotonen-Bildgebung mittels injizierter Fluoreszenzmarker oder vorangegangener Injektion von Fremd-DNA (Transfektion), die zur Bildung von fluoreszierenden Proteinen führt. Gradientenindex-(GRIN-)Linsen mit typischen Durchmessern von 0,2 mm bis 2 mm ermöglichen den Aufbau von miniaturisierten Systemen. Dank ihrer planen Endflächen lassen sich Mehrlinsensysteme einfach und kompakt herstellen. Die NA hängt vom verwendeten Material und dem Herstellungsprozess des Brechzahlgradienten ab. Für silberdotierte GRIN-Linsen beträgt die NA maximal 0,48 (NIR bei 850 nm) und für thalliumdotierte 0,55 (850 nm). Die Auflösung, die Anregungseffizienz und die Detektionseffizienz sind jedoch aufgrund der geringen NA gering.

Um die für eine multiphotonen-basierte Materialabtragung notwendige wesentlich höhere Lichtintensität als im Fall der Diagnostik zu erreichen, würden extrem hohe Laserimpulsenergien erforderlich werden. Dies würde kostenintensive, aufwendige Laserapparaturen erfordern. Zudem würde ein hohes Gefährdungspotenzial bestehen. Eine Bearbeitungspräzision im Sub-100 µm-Bereich innerhalb des Targets könnte infolge des Auftretens kollateraler Schäden, inklusive der Formation großer Kavitätsblasen im Bereich größer als eine Zelldimension sowie unkontrollierter Selbstfokussierung (Nebenschäden korrelieren mit der Impulsenergie), nicht erreicht werden.

Des Weiteren ist in der WO 2006/045936 A2 ein Verfahren und ein System zur fasergestützten multiphotonen-mikroskopischen Abbildung einer Probe offenbart, wobei der Anregungsstrahl mittels mechanischer Spiegelscanner gescannt wird, bevor er in ein Lichtleiterbündel aus vielen einzelnen Fasern zur Übertragung auf eine einheitliche Fokussieroptik eingekoppelt wird. infolge der Strahlablenkung mit Spiegelscanner entstehen unterschiedlich schräge Lichtfallswinkel für alle außerhalb der optischen Achse liegenden Einzelfasern und die folgende Optik, die Signalverfälschungen bei der Abbildung der Objektpunkte zur Folge haben und eine geometrisch hochgenaue Bildauflösung im Submikrometerbereich verhindern.

Ferner wird in der WO2006/004743 A2 ein optischer Faserscanner für eine Multimode-Bildaufnahme beschrieben, bei dem das objektfeld durch eine abtastende Faser in Resonanz, d.h. dynamisch gescannt wird. Der Nachteil dieses Scanners besteht darin, dass eine gezielte systematische Auswahl eines Objektbereiches, eine systematische Punktabtastung oder punktweise Bearbeitung damit nicht möglich sind. Insbesondere ist die Anwendung des freischwingenden Faserendes für eine endoskopische Anwendung mit direkten Objektkontakt nicht möglich.

Alle genannten Verfahren und Anordnungen haben den Nachteil, dass sie keinen endoskopischen Einsatz von Multiphotonen-Prozessen mittels Strahlung eines Femtosekundenlasers im Inneren von Materialien und im Körperinneren für eine präzise Bildgebung und/oder Bearbeitung mit einer Genauigkeit von weniger als einem Millimeter ermöglichen.

Die Entwicklung mehrerer mikroskopischer optischer Abbildungsverfahren (sog. Mikro-Imaging), wie Laser-Scanning-Mikroskopie (LSM), Optisch kohärente Tomographie (OCT) und Multiphotonen-Imaging (MPI) hat das optische Mikro-Imaging revolutioniert und seine Möglichkeiten schnell erweitert (siehe z.B. Concello et al. in: Nature Methods 2 (12), 2005, S. 920-931; Helmchen et al., a.a.O., S. 932-940). Die auf diesen Verfahren basierenden Geräte werden zunehmend im biomedizinischen Bereich eingesetzt, weil sie mikroskopische Untersuchungen schnell, nicht invasiv und ohne Benutzung von Kontrastsubstanzen erlauben (siehe z.B. Flushberg et al., a.a.O., S. 941-950).

Zurzeit werden die optischen mikroskopischen Abtastverfahren meist für externe bzw. oberflächennahe Untersuchungen eingesetzt, weil flexible und miniaturisierte Geräte mit Scanning-Verfahren an der fehlenden Miniaturisierung der Scanner scheitern.

Vorhandene optisch abbildende Lösungen, wie z.B. LSM-Endoskope gemäß EP 1 468 322, haben nur eine beschränkte Auflösung und können ausschließlich für LSM verwendet werden. MPI-Endoskope (z.B. Ling Fu et al. in: Optics Express 14 (3), 2006, S. 1027-1032) haben intrinsisch relativ große Abmessungen; da ein MEMS-Spiegel-Scanner (micro-electromechanical system) mit guten optischen Parametern nicht kleiner als ca. 3 mm ist (wodurch ein Endoskop mit MEMS im inneren Durchmesser nicht kleiner als ca. 5 mm sein kann).

Der Erfindung liegt die Aufgabe zugrunde, eine neue Möglichkeit zur endoskopischen Anwendung von Multiphotonen-Prozessen zu finden, die eine präzise Bildgebung und/oder Mikrobearbeitung von Materialien, insbesondere von biologischen Materialien, mit einer Genauigkeit von weniger als einem Millimeter mittels der Strahlung eines Femtosekundenlasers gestattet. Es ist eine weitere Aufgabe der Erfindung, eine Möglichkeit zur mikroskopisch abtastenden optischen Abbildung, insbesondere für flexible medizinische Handgeräte und Mikroendoskope, zu finden, die eine Miniaturisierung und Flexibilisierung eines optischen Kopfes bei hoher örtlicher Auflösung gestattet, ohne an ein bestimmtes Abbildungs- und Analyseverfahren (z.B. MPI, OCT oder LSM) gebunden zu sein.

Eine erweiterte Aufgabe besteht in der flexiblen Anwendung des miniaturisierten optischen Kopfes für mikroskopische Bearbeitung von biologischem Gewebe und anderen Materialien sowie für kombinierte Abbildungs- und Bearbeitungsgeräte.

Erfindungsgemäß wird die Aufgabe bei einem Verfahren zur Erzeugung mikroskopisch hochaufgelöster Abbildungen auf Basis von lasergenerierter Objektreaktionsstrahlung, durch folgende Schritte gelöst:
- gepulstes Einstrahlen fokussierter Anregungsstrahlung von einem Lasersystem in ein Objekt mittels einer Transmissions-Fokussier-Optikeinheit, bestehend aus einem Transmissionssystem und einer mit dessen Ende starr verbundenen Miniatur-Fokussieroptik hoher numerischer Apertur größer 0,55, zur Auslösung einer lokalen Reaktiansstrahlung des Objekts im Mikrometer- bis Nanometerbereich,
- mindestens zweidimensionale Scanbewegung des distalen Endes der Transmissions-Fokussier-Optikeinheit mit der starr verbundenen Miniatur-Fokussieroptik durch in x- und y- Richtung geordnetes Bewegen des distalen Endes mittels eines Scan-Aktuators innerhalb eines Gehäuses eines typischen endoskopischen Handteils über einem optischen Fenster, zum sukzessive örtlich veränderten Positionieren der Anregungsstrahlung gegenüber dem am Fenster anliegenden Objekt und örtlich zugeordneten hochaufgelösten Abtasten von Objektreaktionsstrahlung und
- Übertragung von örtlich geordnet fortschreitend abgetasteter Objektreaktionsstrahlung mittels der Transmissions-Fokussier-Optikeinheit an ein bildgebendes System mit Photonendetektor.

Vorteilhaft erfolgt ein dritter zu der zweidimensionalen Scanbewegung orthogonaler Scanvorgang durch axiales Bewegen der Transmissions-Fokussier-Optikeinheit zur Veränderung der Tiefe einer aus dem zweidimensionalen Scanbewegung erzeugten Bildaufnahme der Objektreaktionsstrahlung in einer durch die Miniatur-Fokussieroptik vorgegebenen Fokusebene.

Die Miniatur-Fokussieroptik und eine zu dem Laser- und dem bildgebenden System gehörige Mikrooptik, die eine direkte optische Kopplung zu dem Laser- und dem bildgebenden System herstellt, werden zweckmäßig mittels einer starren Verbindung als Transmissions-Fokussier-Optikeinheit gleichzeitig scannend bewegt, wobei in axialer Richtung eine Relativbewegung zur Einstellung der Tiefe der Fokusebene im Objekt vorgenommen werden kann. Dabei wird vorzugsweise die Fokusebene durch Variation des Abstandes zwischen Transmissionssystem und Miniatur-Fokussieroptik variiert.

In einer bevorzugten Variante werden die Miniatur-Fokussieroptik und eine optische Faser, die eine optische Kopplung zu dem Laser- und dem bildgebenden System herstellt, mittels einer starren Verbindung als Transmissions-Fokussier-Optikeinheit gleichzeitig scannend bewegt. Dabei kann durch eine Art Faserbündel aus mehreren Faser-Fokussier-Optikeinheiten simultan eine mehrkanalige Abtastung des Objekts durchgeführt werden oder es können Abbildungs- und Bearbeitungsfunktionen des Objekts quasi-simultan vorgenommen werden, indemdie Anregungsstrahlung durch Parameteränderung in einem Kanal zur Gewebebearbeitung eingesetzt wird.

Die erweiterte Aufgabe der Erfindung wird erfindungsgemäß gelöst durch:
- Umschaltung der Anregungsstrahlung zu einer ieistungserhöhten Wirkungsstrahlung,
- gepulstes Einstrahlen der mittels der Transmissions-Fokussier-Optikeinheit fokussierten Wirkungsstrahlung in das Objekt zur Auslösung von lokalen Gewebeveränderungen und
- definierte Bewegung des distalen Endes der Transmissions-Fokussier-Optikeinheit zur Ausführung dreidimensionaler Bearbeitungsschritte im Objekt.

Dabei wird die Strahlung eines NIR-Femtosekundenlasers mittels Miniaturoptiken hoher numerischer Apertur, insbesondere GRIN-Optiken, bevorzugt in Kombination mit flexiblen optischen Lichtleitern, verspiegelten Hohlleitern oder starren stabförmigen GRIN-Optiken nahezu dispersionsfrei ins Objekt übertragen und auf einen Beleuchtungsspot kleiner 100 µm, bevorzugt um 1 µm, auf ein Target fokussiert. In einer Fokusebene kann mit dem Femtosekundenlaser auch biologisches Gewebe bearbeitet werden, wenn die transiente Intensität im Bereich größer 100 GW/cm², typischerweise im Bereich 1-20 TW/cm², liegt.
Durch Verschiebung der Fokusebene, z.B. mittels einer piezogetriebenen Verstelleinheit, kann die Fokusebene variiert werden und so eine Materialbearbeitung in verschiedenen Targettiefen ermöglicht werden. Die Impulsbreite am Target beträgt zweckmäßig weniger als 10 ps, bevorzugt kleiner 400 fs. Wegen der geringen Absorptions- und Streukoeffizienten im NIR-Bereich wird eine NIR-Laserwellenlänge bevorzugt, die Erfindung umfasst jedoch auch Laserwellenlängen im sichtbaren Bereich, insbesondere die durch einen SHG-Kristall erzeugte frequenzverdoppelte Strahlung eines Femtosekundenlasers sowie Strahlung im UV-Bereich, insbesondere die THG-Laserstrahlung.
Es können Laserimpulse mit Folgefrequenzen im MHz-Bereich, aber auch mit geringerer Folgefrequenz (z.B. im kHz-Bereich), sowie Einzelimpulse für die Bearbeitung verwendet werden. Bevorzugt werden Impulse im Nanojoule-Bereich eingesetzt.

Vorteilhaft erfolgt die Umschaltung zur Einstrahlung der leistungserhöhten Wirkungsstrahlung für die Gewebebearbeitung des Objekt im Wechsel mit dem gescannten Einstrahlen leistungsverminderter Anregungsstrahlung zur ortsauflösenden Bildaufnahme von Objektreaktionsstrahlung erfolgt. Die vom Objekt emittierte Strahlung wird zweckmäßig mittels zeitkorrelierter Einzelphotonen-Zählung detektiert.

Die Aufgabe der Erfindung wird ferner mit einem Verfahren zur Mikrobearbeitung von nichtbiologischem Material oder biologischem Material, insbesondere von Hautgewebe, in der Laser-Endoskopie mit einer Präzision von weniger als einem Millimeter, dadurch gelöst, dass Laserimpulse eines Femtosekundenlasers mit einer impulsenergie von 0,05 nJ bis 100 µJ über die Faser in die objektseitig angeordnete Miniatur-Fokussieroptik mit einer hohen numerischen Apertur eingekoppelt und mit einem Beleuchtungsspot von weniger als 10 µm auf ein Target innerhalb des Objekts fokussiert werden, dass die Laserimpulse mit transienten Intensitäten von mehr als 100 GW/cm² Multiphotonenprozesse in Form einer Targetionisierung, eines optischen Durchbruchs und einer Plasmabildung auslösen, die für eine endoskopische Mikrobearbeitung mit einer Genauigkeit unter 100 µm genutzt werden, ohne dass in Nachbarbereichen signifikante kollaterale Schäden auftreten, dass mindestens zweidimensionale laterale Bewegungen der Fokussieroptik innerhalb eines Endoskopkopf-Gehäuses zum sukzessive räumlich veränderten Ausrichten der Anregungsstrahlung gegenüber dem Objekt ausgeführt werden und dass die vom Target ausgesendete Plasmastrahlung sowie weitere emittierte Objektreaktionsstrahlung bildgebend mittels einer Bestrahlungs- und Detektionseinrichtung, vorzugsweise eines Multiphotonen-Lasermikroskops, detektiert wird, um die Mikrobearbeitung zu überwachen.

Dadurch können durch eine Einzelpunktbeleuchtung einzelne Zellen innerhalb eines Gewebeverbandes optisch zerstört werden, ohne benachbarte Zellen zu schädigen, wobei der Durchmesser des zerstörten Bereichs durch Wahl der Expositionszeit sowie der verwendeten Laserimpulsenergie variiert wird. Bei gleicher Konditionierung kann durch lineare Bewegung ein präziser Schnitt mit Schnittbreiten kleiner 10 µm im Inneren eines Objekts ausgeführt werden, wobei die Schnittbreite durch Wahl von Expositionszeit und Laserimpulsenergie variiert wird.

Wird ein interessierender Bereich (region of interest - ROI) systematisch abgescannt, kann ein präziser Materialabtrag auf größeren Flächen von 100 µm² bis 1 mm² mit einer Präzision von weniger als 10 µm und einer Tiefenwirkung von weniger als 20 µm realisiert werden. Bei zusätzlicher Verschiebung der Fokusebene ist es möglich, einen großvolumigen Materialabtrag im Bereich 1 µm³ bis 1000 mm³ zu realisieren.
Die vom Target ausgesendete Strahlung während der Laserbearbeitung, insbesondere das Plasmaleuchten, kann über die gleiche Strahlführung wie die Laserstrahlung (flexible mikrostrukturierte Faser, verspiegelter Hohlleiter, GRIN-Optik) und die Fokussieroptik, aufgenommen, transportiert und mittels Strahlteiler für eine Detektion ausgekoppelt werden. Zudem kann bei Intensitäten unterhalb der Schwelle für den optischen Durchbruch, typischerweise im Bereich von 1-100 GW/cm² durch Abrastern des Objektes eine Bildaufnahme durch Detektion der Zwei- und Dreiphotonen-Fluoreszenz sowie der SHG und THG von bestimmten Objektstrukturen, z.B. Kollagenfasern, erzielt werden Dies ermöglicht das genaue Auffinden eines zu bearbeitenden Targets. Oftmals kann die SHG und die Autofluoreszenz ohne zusätzliche Objektmarkierung für die Targetsuche genutzt werden. Diese Bildgebung kann außerdem genutzt werden, unmittelbar nach der Bestrahlung mit intensiven Laserimpulsen im TW/cm²-Bereich, den Effekt der Laserbearbeitung bildgebend darzustellen. Interessanterweise wurde in Vorversuchen beobachtet, dass bearbeitetes biologisches Material eine intensive Lumineszenz im Bereich der Laserbearbeitung emittiert.

Des Weiteren wird die Aufgabe bei einem miniaturisierten mikroskopischen Kopf für endoskopische Anwendungen, bei dem eine Transmissionsoptik zur Zuführung einer Anregungsstrahlung, eine Fokussieroptik zum gebündelten Energieeintrag der Anregungsstrahlung in ein Objekt, und eine Scaneinrichtung zum Ändern des Ortes des Energieeintrags in einem Gehäuse angeordnet sind, wobei die Fokussieroptik durch ein Fenster im Gehäuse die Wirkungsstrahlung in das Objekt fokussiert, dadurch gelöst, dass die Fokussieroptik eine Miniatur-Fokussieroptik ist, die einen Durchmesser von weniger als 2 mm und eine numerische Apertur von NA > 0,55 aufweist, wobei die Anregungsstrahlung mittels der Miniatur-Fokussieroptik lokal auf einen Bereich von weniger als 100 µm begrenzt in das Objekt fokussiert ist, dass die Transmissionsoptik mit ihrem in einem typischen Endoskopkopf-Gehäuse (mit 5-6 mm Durchmesser) befindlichen Ende starr mit der Miniatur-Fokussieroptik verbunden ist und eine Transmissions-Fokussier-Optikeinheit bildet, und dass die Scaneinrichtung mindestens einen in einer Ebene zweidimensional beweglichen Scan-Aktuator zur lateralen Bewegung der Miniatur-Fokussieroptik in unmittelbarer Nähe des Fensters des Endoskopkopf-Gehäuses aufweist.

Zweckmäßig weist die Scaneinrichtung weiterhin eine axial bewegliche Verstelleinheit zur axialen Bewegung der Transmissions-Fokussier-Optikeinheit auf, wobei die Transmissions-Fokussier-Optikeinheit in der Verstelleinheit axial fest eingespannt ist.

Dabei ist die Transmissions-Fokussier-Optikeinheit in einer ersten Variante aus der Miniatur-Fokussieroptik und einer optischen Faser starr zusammengesetzt, wobei die Transmissions-Fokussier-Optikeinheit in der Verstelleinheit axial fest eingespannt ist. in einer zweiten Variante ist anstelle der optischen Faser ein verspiegelten Hohlleiter eingesetzt.

In einer dritten Variante besteht sie aus der Miniatur-Fokussieroptik und einer Mikroskopoptik einer Bestrahlungs- und Detektionseinrichtung, wobei der zweidimensionale Scan-Aktuator und die axiale Verstelleinheit ausschließlich an die Miniatur-Fokussieroptik gekoppelt sind.

Die Miniatur-Fokussieroptik ist vorzugsweise eine mehrlinsige GRIN-Optik, die entweder eine gerundete Endfläche aufweist oder mit einem vorgeordneten refraktiven Kugellinsensegment versehen ist, um die hohe numerische Apertur von NA > 0,55 (bis zu NA = 0,85) zu erreichen.

Um die axiale Auflösung noch weiter zu verbessern, enthält die Fokussieroptik zweckmäßig eine zweilinsige GRIN-Optik und eine dazwischen angeordnete diffraktive Optik.

Vorteilhaft ist in der Scaneinrichtung der zweidimensionale Scan-Aktuator als Piezo-Scanner ausgebildet. Er kann aber auch zweckmäßig als elektrostatischer Scanner oder als elektromagnetischer Scanner ausgebildet sein.
Für die Realisierung eines 3D-Scanners ist die axiale Verstelleinheit vorteilhaft mit dem zweidimensionalen Scan-Aktuator verbunden und der Scan-Aktuator in einer axial beweglichen Scannerhalterung befestigt.

Die Transmissionsoptik ist vorteilhaft eine mikrostrukturierte photonische Kristallfaser (PCF), weil diese die Femtosekunden-Laserstrahlung nahezu dispersionsfrei überträgt. Für ortsaufgelöste Bildaufnahmen wird vorzugsweise eine Faser vom Typ Double-Clad-Large-Area-Core-PCF eingesetzt. Für andere Anwendungen ist die Faser als eine Large-Area-Core-PCF ausgebildet.

Vorteilhaft ist im Scannergehäuse um die Faser herum mindestens ein Photoempfänger zum direkten Aufnehmen der Objektreaktionsstrahlung angeordnet. Der Photoempfänger ist vorzugsweise mit einer optischen Filterkombination versehen.
Zweckmäßig können im Gehäuse um die Faser herum mehrere Photoempfänger zum direkten Aufnehmen unterschiedlicher Bestandteile von Objektreaktionsstrahlung angeordnet sein.

Um eine Mehrkanalabtastung zu erreichen, sind im Gehäuse vorteilhaft mehrere Fasern in einer Faserhalterung parallel geführt. Dabei sind die Fasern zweckmäßig in einer flexiblen Bandage eingebettet und weisen Versteifungselemente zur Schwingungsunterdrückung auf.
Das Gehäuse ist vorzugsweise rohrförmig ausgebildet und ist an dessen Stirnseiten durch einen Deckel und ein Fenster hermetisch abgedichtet.
Insbesondere für endoskopische Anwendungen weist das Gehäuse mindestens eine ebene Seitenwand auf, wobei in der ebenen Seitenwand des Gehäuses seitlich ein Fenster angebracht und das Gehäuse mit stirnseitigen Deckeln hermetisch abgedichtet ist.

Für diesen Aufbau enthält die Miniatur-Fokussieroptik ein strahlungsumlenkendes Element, so dass die Anregungsstrahlung lateral aus der Transmissions-Fokussier-Optikeinheit austritt und durch das seitlich angeordnete Fenster auf das Objekt fokussierbar ist, wobei die axiale Verstelleinheit für eine erste laterale Scan-Dimension am Objekt und der Scan-Aktuator für eine zweite laterale Scan-Dimension sowie den Tiefenscan im Objekt vorgesehen sind.
Das hermetisch abgedichtete Gehäuse ist zweckmäßig evakuiert, um eine ungehinderte Bewegung des distalen Endes der Transmissions-Fokussier-Optikeinheit zu gewährleisten.

Die Erfindung basiert auf der Überlegung, dass herkömmliche endoskopische Bildaufnehmer oder mikrochirurgische Einheiten entweder eine zu geringe optische Auflösung oder zu große Abmessungen aufweisen oder aber an ein bestimmtes mikroskopisches Verfahren zur Abbildung gekoppelt sind, so dass biologische Gewebe nur anhand bestimmter Eigenschaften abgebildet werden können. Die Geräte sind auch schwer mit bearbeitenden optischen Anordnungen kombinierbar.
Ferner haben sich endoskopische Bildaufnehmer mit GRIN-Optiken durchgesetzt, bei denen der weiteren Miniaturisierung entgegensteht, dass der Scanablenkungswinkel und somit die laterale Auflösung sehr begrenzt sind. Außerdem sind die optischen Köpfe jeweils für nur eines der mikroskopischen Abbildungsverfahren (MPI, LSM u.a.) tatsächlich angepasst und anwendbar.

Die Erfindung löst dieses mannigfaltige Problemgefüge, indem der bisher nicht ausreichende Scanwinkel der Strahlung vor einer Fokussieroptik durch 2D- oder 3D-Scannen einer mit der Lichtleitfaser starr verbundenen Fokussieroptik realisiert wird, wobei die fasergekoppelte Fokussieroptik als integrales Teil durch einen Aktuator über ein dünnes optisches Fenster gescannt wird. Durch diese Fokussieroptik wird die Wirkungsstrahlung (d.h. die Anregungsstrahlung für eines der oben genannten bildgebenden Verfahren) aus der Faser ausgekoppelt, mittels der Fokussieroptik in einen Punkt des Objekts fokussiert und vom Objekt zurückgeworfene Reaktionsstrahlung - wiederum von der Fokussieroptik gebündelt und über das proximale Ende der Fokussieroptik an die numerische Apertur der Lichtleitfaser angepasst einem Bildsensor zugeleitet. In Abhängigkeit von dem eingesetzten Imaging-Verfahren werden unterschiedliche Fokussieroptiken (für MPI oder LSM z.B. mit hoher distaler numerischer Apertur (IVA > 0,5) verwendet.
Die zum Einsatz kommenden Aktuatoren, die ein 2D-Scanningmuster ermöglichen, sind z.B. miniaturisierte Piezo-, elektrostatische oder elektromagnetische Aktuatoren. Der Abstand des distalen Endes der Fokussieroptik vom Fenster und die Fensterdicke werden mit dem Arbeitsabstand der Fokussieroptik (d.h. ihrer distalen Fokallänge) abgestimmt und berücksichtigen Änderungen des Abstandes vom Fenster für die Tiefenverstellung der fasergekoppelten Fokussieroptik. Beim lateralen Scannen des Faser-Fokussieroptik-Verbundes werden Punkte einer vorgewählten Ebene des Objekts systematisch fortschreitend abgetastet. Die nach Einwirkung der Wirkungsstrahlung erzeugte Objektreaktionsstrahlung, wie z.B. Fluoreszenz oder SHG (Second Harmonic Generation), wird über die Fokussieroptik und eine Lichtleitfaser an eine Sensoreinheit übertragen.

Die zweite Teilaufgabe, die Flexibilisierung der Erfindung für die Anwendung verschiedener Abbildungsverfahren, wird erzielt, indem die (optische) Wirkungsstrahlung einer Lichtquelle durch eine flexible Lichtleitfaser zum abzubildenden Objekt transportiert und dort zur scannenden Abtastung des Objekts verwendet und die Reaktionsstrahlung wieder vom Lichtleitfaser aufgenommen wird (sog. "Direkt-Scanner"). In Abhängigkeit von einer eingesetzten Imaging-Verfahren sind unterschiedliche optische Fasern (Transportfasern) erforderlich, für das MPI benutzt man z.B. PCF (Photonic Crystal Fibers). Der Abtastkopf ist somit durch Ankopplung verschiedener Fasern mit anderen Abbildungsverfahren anwendbar.
Durch die Faser wird eine Wirkungsstrahlung (bzw. Anregungsstrahlung) zum Objekt geleitet und von der Fokussieroptik auf der Oberfläche oder in der Tiefe des Objekts fokussiert sowie eine vom Objekt zurückkommende Reaktionsstrahlung durch das optische Fenster über die Fokussieroptik einer externen oder unmittelbar im Scannergehäuse befindlichen Sensoreinheit zugeführt.
Ein so beschaffener Direkt-Scanner kann eine hohe axiale und laterale Auflösung der Objektabbildung im Mikrometerbereich realisieren. Zusätzlich kann der Scanner in Mikro- und Nanochirurgie sowie für Bearbeitung verschiedener Gewebe und Stoffe als geführtes Bearbeitungswerkzeug eingesetzt werden, indem die Wirkungsstrahlung durch eine wesentlich erhöhte Leistung für Laser-Schneid- bzw. Bearbeitungsvorgänge verwendet und alternativ zur Beobachtung umgeschaltet wird.

Mit der erfindungsgemäßen Lösung werden neue endoskopische Anwendungen von Multiphotonen-Prozessen ermöglicht, die eine präzise Bildgebung und/oder Mikrobearbeitung von Materialien, insbesondere von biologischen Materialien, mit einer Genauigkeit von weniger als einem Millimeter mittels der Strahlung eines Femtosekundenlasers gestatten. Ein miniaturisierter mikroskopischer Aufnahmekopf, insbesondere für flexible medizinische Handgeräte und Mikroendoskope, ermöglicht dabei für verschiedene bildgebende Abbildungsverfahren, vorzugsweise für Multiphotonen-Imaging (MPI) und Laser-Scanning-Mikroskopie (LSM), mit wesentlich kleineren, leichteren und flexibleren Messköpfen hochaufgelöst abgetastete Abbildungen zu erhalten sowie mit derselben Einrichtung das MPI-Verfahren (z.B. in der Endoskopie) für Mikrobearbeitungsverfahren zu erschließen und mit anderen abbildenden Verfahren zu kombinieren.

Die Erfindung soll anhand von Ausführungsbeispielen näher erläutert werden. Die Zeichnungen zeigen:
- Fig. 1:: einen Prinzipaufbau eines Multiphotonenmikroskops mit sehr hoher numerischer Apertur zur Femtosekundenlaser-Bearbeitung und Beobachtung von Hautgewebe,
- Fig. 2a:: einen Direkt-Scanner mit Piezo-Aktuator für axiales Scanning und Fluoreszenz-Erfassung durch eine Lichtleitfaser (Double-Clad-Faser),
- Fig. 2b:: einen Direkt-Scanner mit Piezo-Aktuator für laterales Scanning, Fluoreszenz-Erfassung durch eine Lichtleitfaser (Double-Clad-Faser),
- Fig. 3:: einen Direkt-Scanner nach Fig. 2a mit einem elektrostatischen Aktuator,
- Fig. 4:: einen Direkt-Scanner nach Fig. 2a mit einem elektromagnetischen Aktuator und Permanentmagnet auf der Faseranordnung,
- Fig. 5:: einen Direkt-Scanner nach Fig. 2a mit einem elektromagnetischen Aktuator und induktiven Elementen auf der Faseranordnung,
- Fig. 6:: einen Direkt-Scanner mit einem elektrostatischen Aktuator, einem unmittelbar im Scanner befindlichen Empfänger (mit Faser-Durchgang) und einer reflektierenden Schicht an den Gehäusewänden,
- Fig.7:: einen Direkt-Scanner mit einem elektrostatischen Aktuator und zwei unmittelbar integrierten Empfängern,
- Fig. 8:: einen Direkt-Scanner mit einem elektromagnetischen Aktuator und einem unmittelbar integrierten Empfänger (ohne Faser-Durchgang)
- Fig.9:: einen Direkt-Scanner mit einem elektromagnetischen Aktuator und Fluoreszenz-Erfassung durch eine Faser mit Breitfeld-Abbildungsanordnung (ohne Faser-Durchgang),
- Fig. 10:: eine Darstellung einer Faser vom Typ Double-Clad-Large-Area-Core-PCF,
- Fig. 11:: eine schematische Darstellung einer Mehrkanalanordnung in einem Gehäuse.

Das erfindungsgemäße Verfahren soll im Folgenden - ohne Beschränkung der Allgemeinheit für die Anwendung anderer lasergestützter Abbildungsverfahren - unter Verwendung eines Femtosekunden-Lasermikroskops, wie es für Ein-, Zwei-und Multiphotonen-Mikroskopie verwendet wird, erklärt werden.

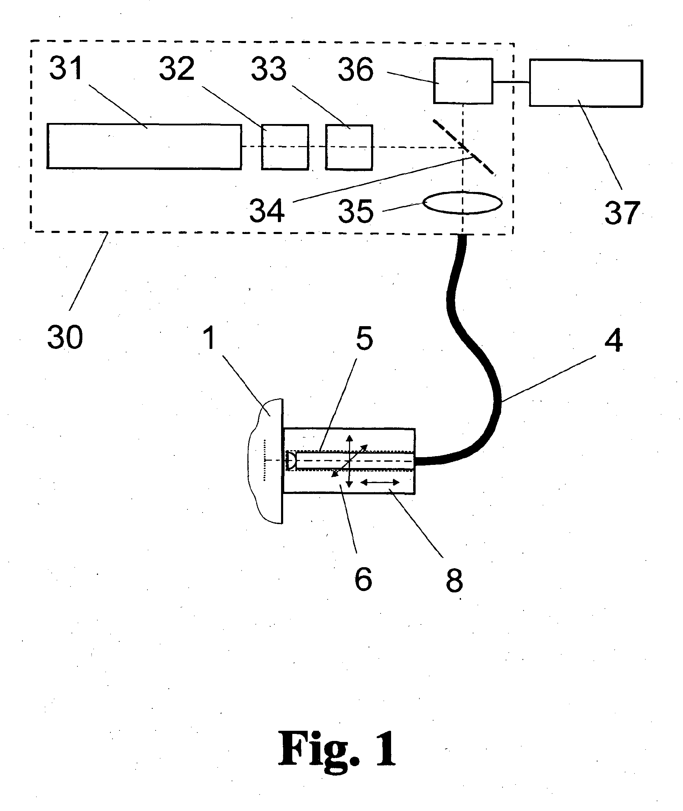

Die Anordnung besteht - wie in Fig. 1 dargestellt - aus einer Bestrahlungs- und Detektionseinrichtung 30, bei der von einem 80 MHz-Titan-Saphir-Femtosekundenlaser 31 gepulste Strahlung einer Emissionswellenlänge von 780 nm erzeugt, durch einen Shutter 32, einen Strahlabschwächer 33 und einen dichroitischen Teilerspiegel 34 übertragen und mittels einer Mikroskopoptik 35 fokussiert bereitgestellt wird und bei dem eine durch die Laseranregung von einem Objekt 1 zurückkommende Sekundärstrahlung (z.B. Ein-, Zwei- oder Multiphotonen-Fluoreszenz, SHG, THG, Lumineszenz etc.) über den Teilerspiegel 34 auf einen Photonendetektor 36 ausgekoppelt wird.

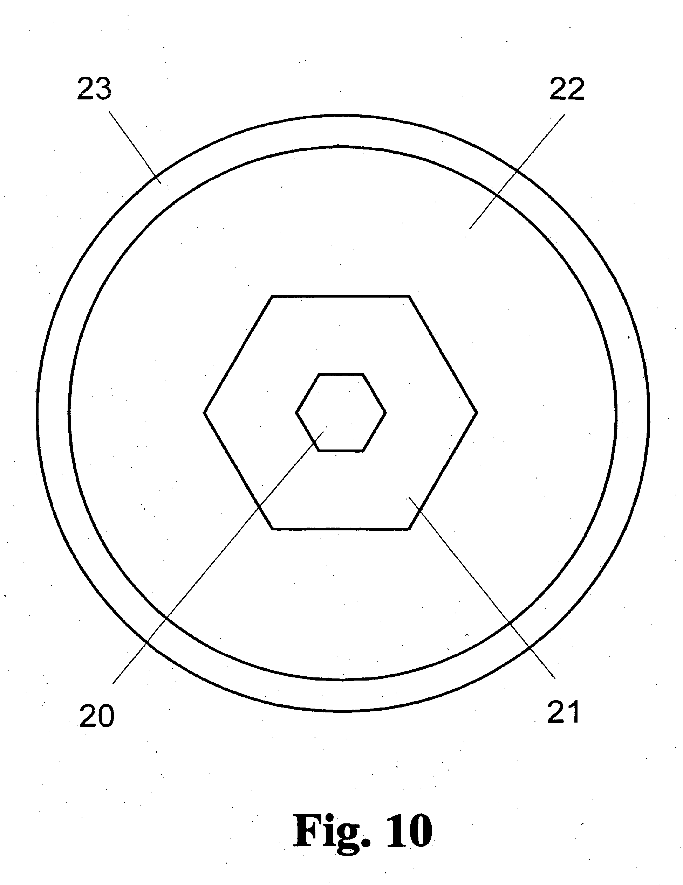

Das Mikroskopobjektiv 35 der Bestrahlungs- und Detektionseinrichtung 30 koppelt die Laserstrahlung in den zentralen Bereich 20 (nur in Fig. 10 gezeigt) einer optischen Multimode-Faser 4 ein, die als mikrostrukturierte photonische Kristallfaser (PCF), vorzugsweise als Doppelclad-Faser, ausgebildet ist und die Strahlungsübertragung zu einer Miniatur-Fokussieroptik 5 mit hoher numerischer Apertur (NA > 0,55) übernimmt.
Die am distalen Ende der Faser 4 starr angebrachte Miniatur-Fokussieroptik 5 ist eine spezielle Miniatur-GRIN-Optik, bestehend aus Stablinsen mit radialem Gradienten-Brechungsindex sowie gekoppelt mit refraktiven Linsenformen zur Erzielung einer hoher numerischen Apertur (NA), um eine Fokussierung auf einen Bestrahlungsspot kleiner 1 µm zu erreichen. Zu diesem Zwecke ist die distale Linse in Form einer Halbkugel geformt oder der GRIN-Optik wird ein refraktives Halbkugellinsensegment 5' vorgeordnet.
Typische Abmessungen dieser speziellen Miniatur-Fokussieroptik 5 sind 1,7 mm Außendurchmesser und eine Länge um 2 cm.

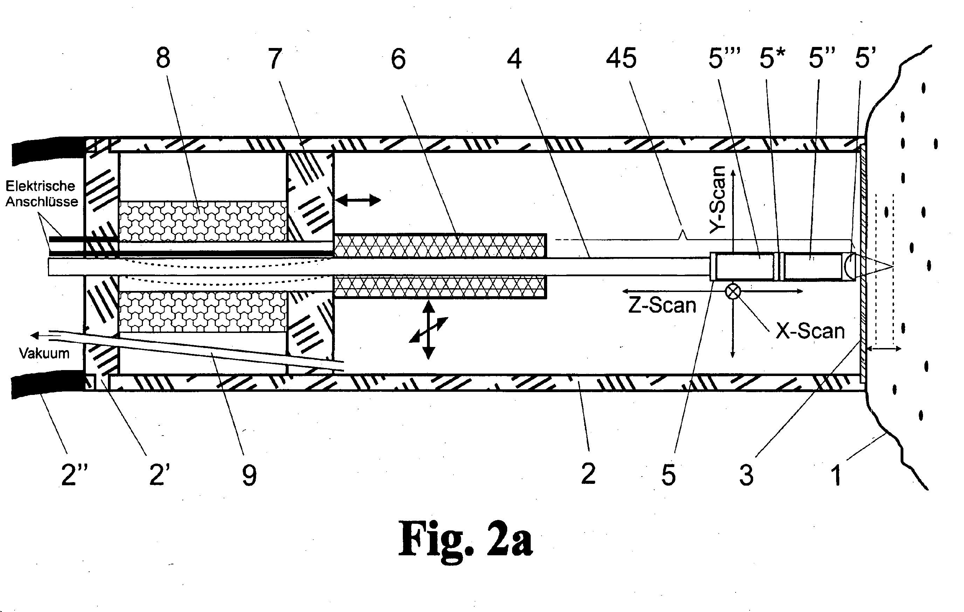

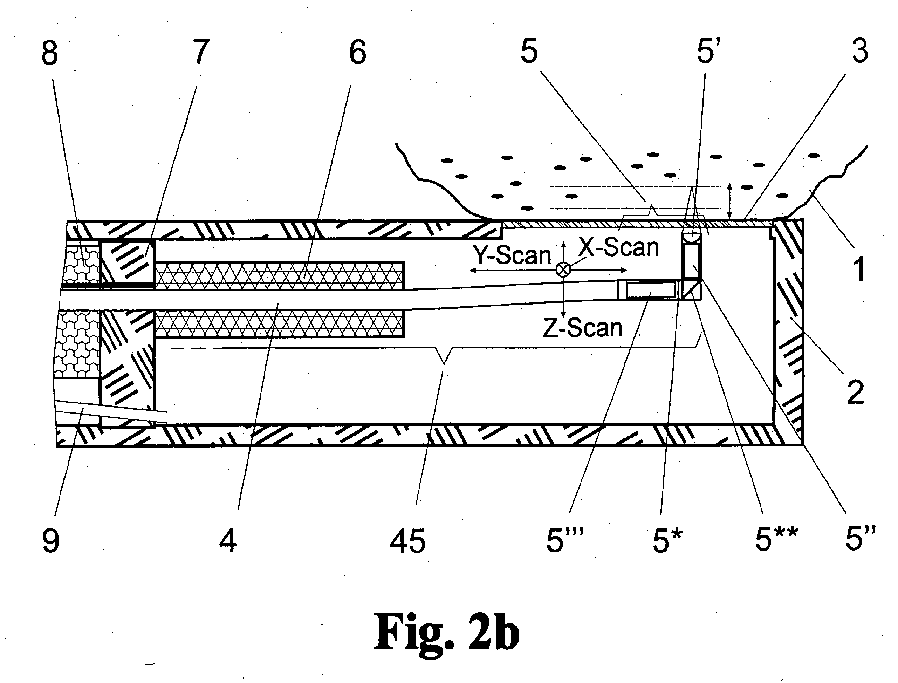

Durch die Miniatur-Fokussieroptik 5 werden NIR-Laserimpulse nach Betätigung des Shutters 32 zunächst mit einer über den Abschwächer 33 verringerten Impulsenergie von 0,5 nJ auf das zu bearbeitende Objekt 1 appliziert. Mittels einer Scaneinheit 6, vorzugsweise in Form eines Piezo-Aktuators 6' (gemäß Fig. 2), zur kontrollierten x-y-Auslenkung (Vibration) sowie einer axialen Verstelleinheit 8 zur Variation der Fokusebene, können Flächen in unterschiedlichen Gewebetiefen abgerastert werden.
Vom Objekt 1 ausgesendete Signale in Form von SHG-Strahlung und Zweiphotonen-Fluoreszenz werden von der Miniatur-GRIN-Optik 5 erfasst, vorwiegend im äußeren Clad einer PCF-Faser 4 über die Mikroskopoptik 35 zum dichroitischen Teilerspiegel 34 geleitet und von dort auf einen Photonendetektor 36, typischerweise einem Photomultiplier (PMT), übertragen.
Durch Verknüpfung der Signale des Photonendetektors 36 mit der x-y-Scanposition und der Fokusebene, lässt sich mittels Bildverarbeitung an einem Computer 37 (z.B. PC) eine dreidimensionale Abbildung des Objektes 1 erstellen. Anhand dieser Abbildung kann ein Target innerhalb des Objektes 1 definiert werden. Der Laserstrahl wird auf das Target positioniert und ist nach dem Einstellen einer hohen Impulsenergie, z.B. von 3 nJ, durch Verändern der Transmission des Abschwächers 33 (z.B. durch Positionsänderung) und nach dem Öffnen des Shutters 32 geeignet, in der Fokusebene innerhalb des Objekts 1 ein lokales Plasma zu erzeugen, das zum Bohren (mittels Einzelpunktbeleuchtung - Single-Point-Illumination), zum Schneiden (mittels Line-Scan) sowie zum Inaktivieren einer einzelnen Zelle oder zum Ablatieren durch Scannen eines interessierenden Bereichs (ROI - region of interest) genutzt werden kann.

Während des Bearbeitungsvorganges wird am Photonendetektor 35 ein Signal aus Plasmastrahlung registriert. Nach Beendigung des Bearbeitungsprozesses kann das Objekt 1 erneut mit Laserimpulsen geringer Impulsenergie (z.B. 0,5 nJ) abgerastert werden, um ein Bild anhand des SHG-Signals, der Zweiphotonenfluoreszenz oder der Lumineszenz des bearbeitenden Areals zu gewinnen.

Besonders vorteilhaft wird eine Faser 4 mit einem zentral gelegenen PCF-Lichtleiter zur Übertragung der Laserimpulse und mit peripher liegenden Lichtleitern, die nicht mikrostrukturiert sein müssen, zur Transmission der Objektstrahlung genutzt.
Ferner kann anstelle einer Miniatur-Fokussieroptik 5 mit Kugellinsensegment 5' auch lediglich eine zweilinsige GRIN-Optik zur Erzeugung einer hohen NA eingesetzt werden. In einer weiteren modifizierten Ausführung nutzt man eine Miniatur-GRIN-Optik mit hoher NA durch zusätzliche Krümmung der (sonst üblicherweise planen) distalen Endfläche.

In einer speziellen Anwendung zur Untersuchung der menschlichen Haut wird an einem kommerziellen Zweiphotonen-Mikroskop oder kommerziellen Multiphotonen-Tomographen - beide werden hier unter Bestrahlungs- und Detektionseinrichtung 30 subsumiert - zusätzlich eine Miniatur-Fokussieroptik 5 hoher NA derart starr angekoppelt, dass die durch die Mikroskopoptik 35 der Bestrahlungs- und Detektionseinrichtung 30 (bzw. des Tomographen) definierte Fokusebene mittels der Miniatur-Fokussieroptik 5 in tiefere Schichten des zu bearbeitenden Objektes 1 übertragen wird. So kann beispielsweise mittels stabförmiger 2 cm langer Miniatur-GRIN-Optik 5, die durch eine gekrümmte GRIN-Linsen-Oberfläche oder ein Kugellinsensegment 5' eine sehr hohe numerische Apertur von NA > 0,6 (bis zu 0,85) aufweist, sich in einer speziellen Edelstahlkanüle mit Saphir-Fenster (Fensterdicke kleiner als 200 µm) befindet und an einer dreiachsigen Verstelleinrichtung (Zusammenwirken von lateralem Scan-Aktuator 6 und axialer Verstelleinheit 8) angebracht ist, der Fokus in das Innere des Objekts 1 übertragen werden. Mittels der axialen Verstelleinheit 8 kann der Abstand zur Mikroskopoptik 35 und damit näherungsweise die Fokusebene im Objekt 1 typischerweise in einem Bereich bis zu 0,5 mm mit einer Präzision im SubMikrometerbereich verschoben werden.
Die vom Objekt 1 emittierte Strahlung wird mittels Miniatur-Fokussieroptik 5 aufgefangen und über die Mikroskopoptik 35 und den dichroitischen Teilerspiegel 34 mittels Photonendetektoren 36, die sich innerhalb der Bestrahlungs- und Detektionseinrichtung 30 bzw. Tomographen befinden, detektiert und für eine Bilderzeugung genutzt.
Die Strahlung eines Femtosekundenlasers 31 wird nach Transmission durch Shutter 32, Abschwächer 33 und dichroitischem Teilerspiegel 34 mittels optischem Gelenkarm,
x-y-Galvoscanner und Optik (nicht dargestellt) in einer endoskopischen starren, mittels axialer Verstelleinheit 8 zur Variation der Fokusebene beweglichen Miniatur-Fokussieroptik 5 hoher NA eingekoppelt, die von einem Faserbündel 4 umgeben ist. Die Bearbeitung erfolgt mittels durch die Miniatur-Fokussieroptik 5 transmittierter Strahlung hoher Impulsenergie, während die vom Objekt 1 emittierte Strahlung durch die Miniatur-Fokussieroptik 5 erfasst und durch das umgebende Faserbündel 4 zum Photonendetektor 36 geleitet wird.
Der verwendete Photonendetektor 36 soll sich durch eine schnelle Reaktionszeit auszeichnen, so dass die Ankunft der Photonen der vom Objekt 1 ausgesendeten Strahlung zeitkorreliert, bevorzugt mittels zeitkorrelierter Einzelphotonen-Zählung erfasst werden. Dadurch kann eine zeitliche Auflösung im Bereich weniger Pikosekunden erreicht werden, die zur Ermittlung der Fluoreszenzlebensdauer und zur Separation der SHG/THG- und Plasmastrahlung von der Fluoreszenz genutzt werden kann.

Der Photonendetektor 36 kann ferner als spektraler Detektor ausgebildet sein, indem ein PMT-Array mit einem Polychromator kombiniert wird.

In einer weiteren Gruppe von Ausführungen ist die Anordnung zur hochgenauen Positionierung der Laserstrahlung und Detektion der Objektreaktionsstrahlung auf Basis einer Bestrahlungs- und Detektionseinrichtung 30 als Direkt-Scanner als ein endoskopisch über eine Faser 4 angekoppeltes Handteil realisiert.
Diese Anordnung besteht in ihrem Grundaufbau - wie in Fig. 2a schematisch dargestellt - aus einem handlichen starren Gehäuse 2, das mit einem für die verwendeten optischen Strahlungsanteile transparenten Fenster 3 auf ein Objekt 1 aufgesetzt wird, einer an eine optischen Faser 4 angekoppelte Miniatur-Fokussieroptik 5 sowie einem Scan-Aktuator 6, der die starr verbundene Faser-Fokussieroptik-Anordnung 45 in einem frei wählbaren Scanning-Regime zweidimensional (X-Y-Scan) bewegt. Innerhalb des Gehäuses 2 ist eine axial bewegliche Scannerhalterung 7 vorgesehen, die durch eine axiale Verstelleinheit 8 eine zu den ersten beiden Scan-Richtungen orthogonale dritte Scanbewegung (Z-Scan) ermöglicht. Das Gehäuse 2 ist mit einem Gehäusedeckel 2' abgeschlossen, bevor sich ein flexibler Schlauch 2" für Versorgungsleitungen anschließt, wie z.B. für Fasern 4 (zum Zuführen von Wirkungsstrahlung bzw. leistungsreduzierter Anregungsstrahlung) und Übertragung von Objektreaktionsstrahlung), elektrische Zuleitungen für Scan-Aktuator 6 und Verstelleinheit 8, und optional eine Vakuumleitung 9.
Auf Basis dieser Grundvariante sind verschiedene Betriebsmodi und bauliche Modifikationen realisierbar, die nachfolgend als separate Ausführungsbeispiele beschrieben sind.

### 1. Direkt-Scanner mit 3D-Scan

Der Direkt-Scanner besteht in diesem Beispiel, wie in Fig. 2a gezeigt, aus einer miniaturisierten Fokussieroptik 5, einer Faser 4, mit deren distalem Ende das proximale Ende der Fokussieroptik 5 verbunden ist und einem Scan-Aktuator 6, z.B. Piezo-Aktuator 6', der das distale Ende der Miniatur-Fokussieroptik 5 unmittelbar über einem dünnen optischen Fenster 3, das am Objekt 1 anliegt, abtastet. Der Brennpunkt der Fokussieroptik 5 befindet sich hinter dem Fenster 3 auf der Oberfläche des Objekts 1 oder in seiner Tiefe und wird mit der Miniatur-Fokussieroptik 5 gescannt (x-y-Scan). Die Abtasttiefe im Objekt 1 kann mit einer axialen Verstelleinheit 8 eingestellt werden. Dabei wird die axial bewegliche Scannerhalterung 7, auf der der Scan-Aktuator 6, der die Faser 4 und die Fokussieroptik 5 gemeinsam lateral bewegt, befestigt ist, in der axialen Richtung verschoben. Der Abstand zwischen dem distalen Ende der Fokussieroptik 5 und dem Fenster 3 verändert sich somit und realisiert einen in Längsrichtung des Gehäuses 2 gerichteten Z-Scan. Die freie Länge der Faser 4 innerhalb der axialen Verstelleinheit 8 und der Scannerhalterung 7 wird für den Längenausgleich der Faser 4 bei der Abstandänderung der Faser-Fokussieroptik-Einheit 45 gegenüber dem Fenster 3 verwendet.
Die Miniatur-Fokussieroptik 5 kann einen anderen Durchmesser als die Faser 4 haben und als GRIN-Optik, konventionelle Optik, Fresnel-Optik oder eine Kombination aus GRIN-Optik und anderer Optik (diffraktive Optik, Fresnel-Optik u.a.) ausgeführt sein.

In einer besonders vorteilhaften Ausführung weist die Miniatur-Fokussieroptik 5 ein refraktives Kugellinsensegment 5', dessen distale Fläche eben ist, zwei GRIN-Linsen 5" und 5'" sowie eine dazwischen angeordnete Diffraktivoptik 5* auf. Die objektseitige GRIN-Linse 5" dient zum Ausgleich der Aberrationen des Kugellinsensegments 5' und erzeugt an ihrem proximalen Ende aus der durch das Kugellinsensegment 5' übertragenen stark divergenten Objektreaktionsstrahlung quasi-parallelen oder leicht divergentes Strahlenbündel. Die zweite GRIN-Linse 5"' dient zur Einkopplung dieser Strahlung in die Faser 4 bzw. Auskopplung der Wirkungsstrahlung (Anregungsstrahlung) aus der Faser 4. Die Diffraktivoptik 5* korrigiert chromatische Aberrationen des Kugellinsensegments 5' sowie der GRIN-Linsen 5" und 5"'.

Das Gehäuse 2, das aus einem medizinisch zugelassenen Werkstoff besteht, ist an seinem distalen Ende hermetisch (vakuumdicht) durch das optische Fenster 3 abgeschlossen, das ebenfalls aus medizinisch zugelassenen transparenten Material besteht. An seinem proximalen Ende wird das Gehäuse 2 für medizinische Anwendungen ebenfalls vakuumdicht mit dem Deckel 2' abgeschlossen. Der Deckel 2' ist mit dem Schlauch 2" mit allen für den Scannerbetrieb notwendigen Leitungen verbunden. Auf der distalen Seite des Deckels 2' ist die axiale Verstelleinheit 8 befestigt. Der Deckel 2' gewährleistet die vakuumdichte Durchführung aller zum Scanner führenden Leitungen.
Das Gehäuse 2 kann durch die Vakuumleitung 9 evakuiert werden, um den Luftwiderstand für das distale Ende der Faser-Fokussieroptik-Einheit 45 beim Scannen zu verringern.
Die axiale Verstelleinheit 8 kann als z.B. eine Piezosteller ausgeführt sein.
Der Direkt-Scanner kann somit Punkt-, Linien-, 2D- oder 3D-Scannen in beliebiger Kombination der x, y und z-Bewegungen ausführen, dies wird durch eine gezielte Ansteuerung des 2D-Scan-Aktuators 6 und der axialen Verstelleinheit 8 erreicht.

Fig. 2b zeigt eine spezielle Ausführung des Direkt-Scanners mit einem optischen Fenster 3, das seitlich an einer Wand des Gehäuses 2 angeordnet ist. Die Fokussieroptik 5 beinhaltet in diesem Fall ein Umlenkelement 5** (z.B. ein Prisma), das die Richtung der Strahlung um ca. 90° ablenkt. Dadurch wird die Tiefenverstellung bezüglich des Objekts 1 (z-Scan) bei dieser Bauform des Direkt-Scanners vom Piezo-Aktuator 6' übernommen, der den x-Scan genauso wie in Fig. 2a ausführt. Die axiale Verstelleinheit 8 übernimmt hier den y-Scan.

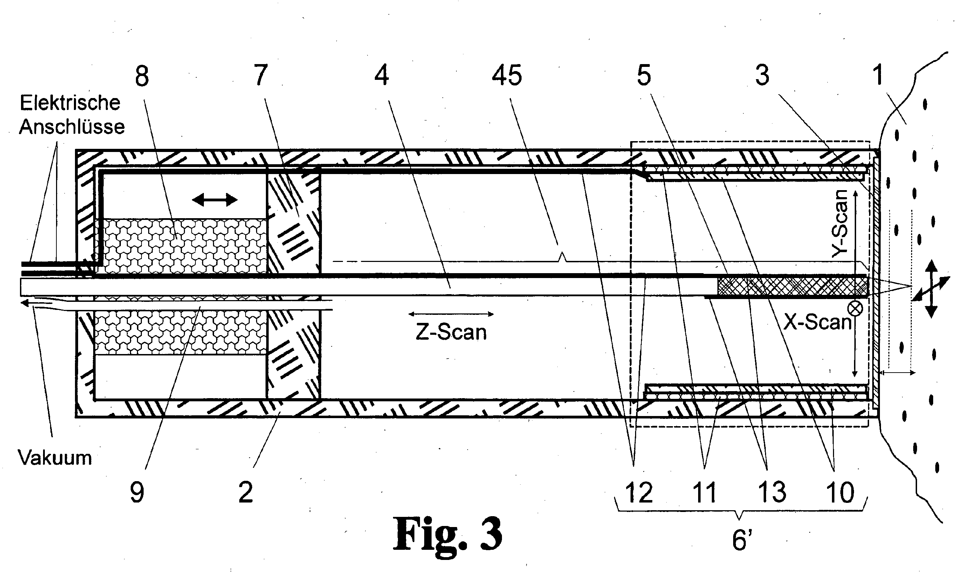

Fig. 3 zeigt eine modifizierte Ausführung des Direkt-Scanners mit einem elektrostatischen Scanner 6". Hier wird die Faser-Fokussieroptik-Einheit 45 dadurch bewegt bzw. verstellt, dass eine leitende Beschichtung 13, die ein elektrisches Potential über die Leitung 12 erhält, durch das elektrische Feld der gegenüberliegenden Schichtelektroden 10 eine gerichtete Wirkung erfährt. Die Schichtelektroden 10 sind unmittelbar auf die Innenwand des Gehäuses 2 aufgebracht, falls das Gehäuse 2 aus einem isolierenden Material besteht oder ruhen auf einer isolierenden Unterlage 11, wenn - wie in Fig. 3 gezeigt - das Gehäuse 2 aus leitenden Material besteht.

Im Übrigen funktioniert der Direkt-Scanner mit dem elektrostatischen Aktuator 6" nach demselben Schema wie mit dem Piezo-Aktuator 6'.

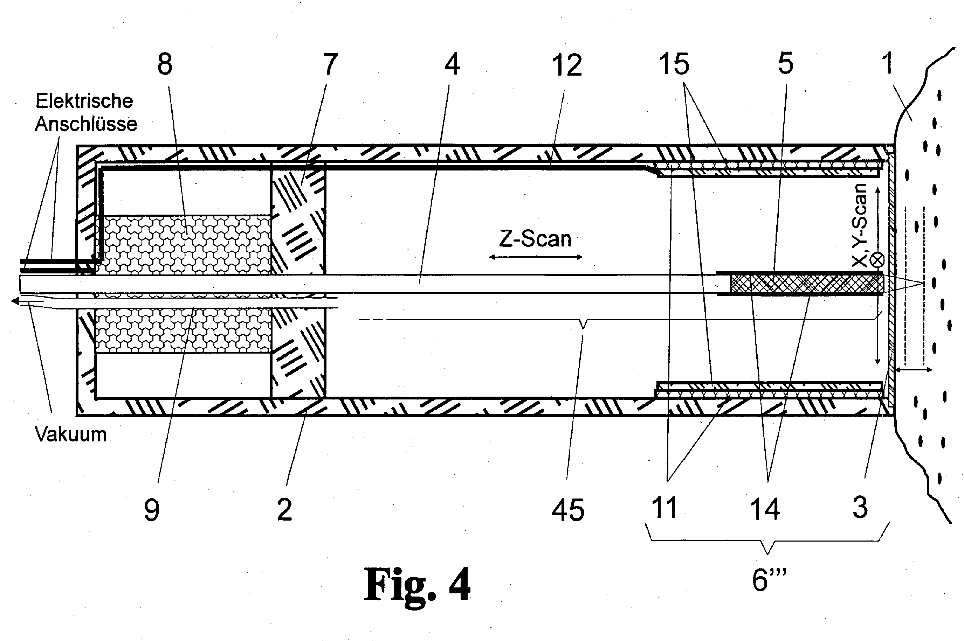

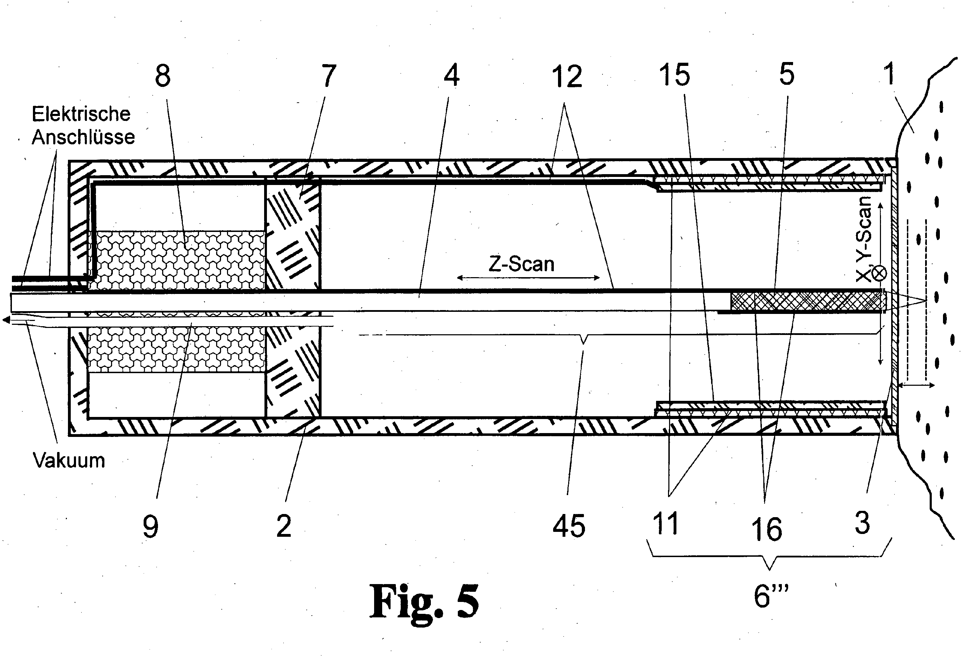

Fig. 4 zeigt eine modifizierte Ausführung des Direkt-Scanners mit einem elektromagnetischen Aktuator 6"', in dem ein Permanentmagnet 14 verwendet wird. Hier wird die Faser-Fokussieroptik-Einheit 45 dadurch bewegt bzw. verstellt, dass der Permanentmagnet 14, der auf die Faser-Fokussieroptik-Einheit 45 (z.B. als ferromagnetische Beschichtung) aufgebracht ist, durch das elektromagnetische Feld der gegenüberliegenden stromleitenden Induktivelemente 15, die an der Innenwand des Gehäuses 2 angebracht sind, eine gerichtete Wirkung erfährt. Falls das Gehäuse 2 aus leitenden Material besteht wird - wie in Fig. 3 - eine isolierende Unterlage in Form einer dielektrischen Schicht 11 verwendet.
Eine weitere Variante dieser Ausführung ist in Fig. 5 dargestellt, bei der der Permanentmagnet 14 durch mehrere Induktivelemente 16 ersetzt ist.
Alle übrigen Funktionen des Direkt-Scanners sind gleich denen der vorhergehenden Ausführungen mit Piezo-Scanner 6' oder elektrostatischem Scanner 6".

Wenn die Ausführungsbeispiele nach den Figuren 2-5 sowie 9 für mikroskopische Abbildungszwecke benutzt werden, wird als Faser 4 eine so genannte Double-Clad-Large-Area-Core-PCF (PCF - Photonic Crystal Fiber) eingesetzt, wie sie nachfolgend mit Bezug auf Fig. 10 beschrieben wird. Eine Faser 4 dieses Typs hat einen zentralen Bereich 20 mit einem gehobenen Brechungsindex n₁, der seine führenden Eigenschaften durch einen mikrostrukturierten Bereich 21 erhält, der von einem Mantel 22 aus einem optischen Material mit Brechungsindex n₂ umgeben ist. Der Mantel 22 ist mit einer äußeren Beschichtung 23 umhüllt, die den niedrigen Brechungsindex n₃ aufweist und vor allem die mechanischen Eigenschaften der Faser 4 verbessert. Im Allgemeinen gilt: n₁ > n₂ > n₃. Der Kernbereich 20 wird zum Zuführen einer Wirkungsstrahlung zum Objekt 1 verwendet. Die im Objekt 1 erzeugte Reaktionsstrahlung wird durch die Miniatur-Fokussieroptik 5 in den Mantel 22 der Faser 4 eingekoppelt, über diese zurückgeleitet und mittels eines Strahlteilers 34 auf einen (externen) Photoempfänger 36 (beide nur in Fig. 1 dargestellt) ausgekoppelt. Der Photoempfänger 36 ist mit notwendigen Farbfiltern und gegebenenfalls mit einer Sammeloptik ausgestattet. In Abhängigkeit von der konkreten Anwendung des Direkt-Scanners kann die Objektreaktionsstrahlung als spiegelnd oder diffus reflektierte Anregungsstrahlung, Photolumineszenz (insbesondere Fluoreszenz), Raman- und Rayleigh-Streuung, erzeugte Harmonische (SHG, THG) der Anregungsstrahlung usw. vorkommen. Der oben genannte Fasertyp ist auch für die Anwendungen vorteilhaft, in denen Abbildung und Gewebebearbeitung kombiniert werden.

Beim Einsatz des Direkt-Scanners ausschließlich für Zwecke der Mikrochirurgie oder Materialbearbeitung ist die Faser 4 vorzugsweise eine sog. Large-Area-Core-PCF. In einer solchen Faser 4 ist der Mantel 22 auf den mikrostrukturierten Bereich 21 reduziert, d.h. der in Fig. 10 gezeichnete Mantel 22 ist als Funktionsschicht völlig entfallen.

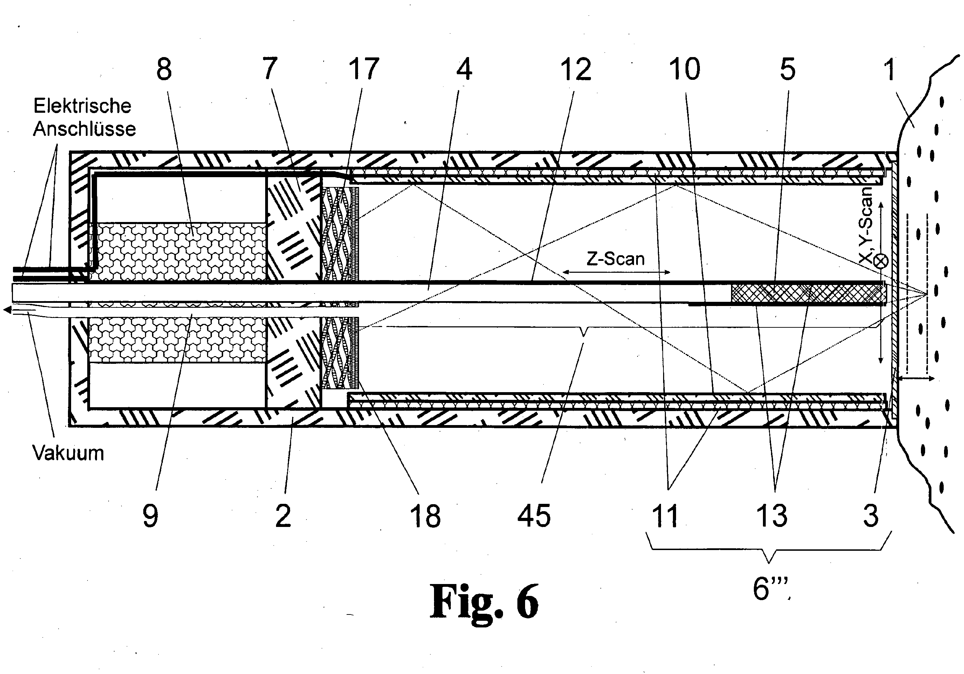

Weitere Möglichkeiten der Erfassung der Objektreaktionsstrahlung sind in den Figuren 6-8 dargestellt, wobei der Direkt-Scanner mit elektrostatischen bzw. elektromagnetischen Aktuatoren 6" bzw. 6'" in spezieller Ausführung arbeitet.
In einer ersten Ausführung gemäß Fig. 6 wird für die Faser 4 eine Large-Area-Core-PCF verwendet. Die durch die Wirkungsstrahlung hervorgerufene Objektreaktionsstrahlung wird innerhalb des Direkt-Scanners von einem Photoempfänger 17 mit vorgeordneter optischer Filterkombination 18, die mindestens aus einem Farbfilter besteht, erfasst. Die Innenwände des Gehäuses 2 auf der distalen Seite des Photoempfängers 17 sind mit einer Spiegelschicht 10 versehen, die ersatzweise auch durch (spiegelnde) Elektroden auf der dielektrischen Schicht 11 gebildet sein kann. Diese Ausführungsvariante ist für miniaturisierte Anwendungen vorteilhaft, in denen der verwendete Photoempfänger 17 ein zentrales Loch für die Faser 4 hat.

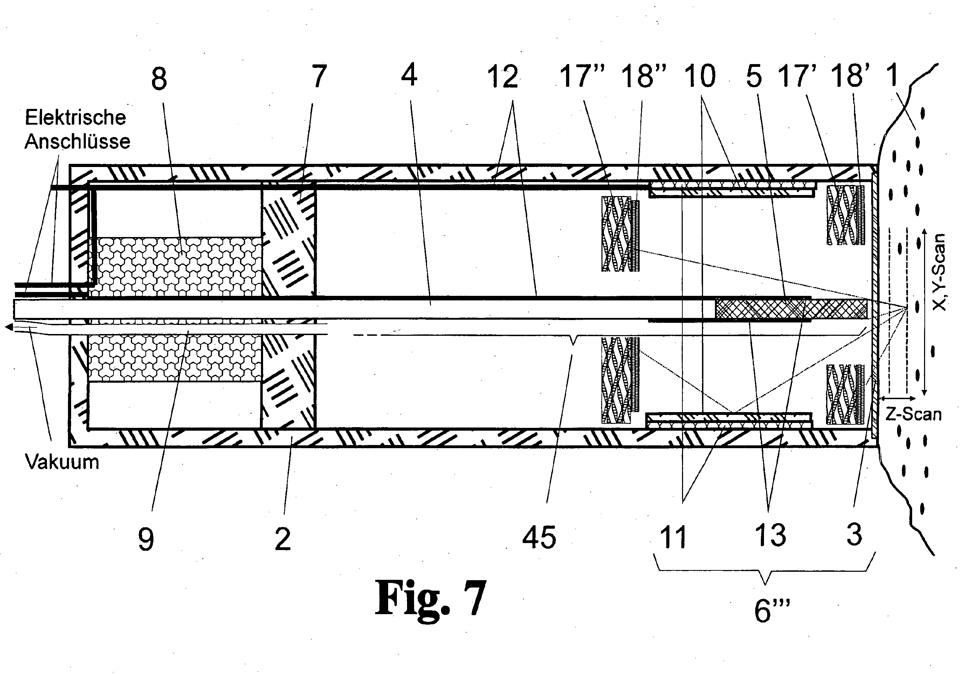

Fig. 7 offenbart eine Ausführung, in der mindestens zwei Photoempfänger 17' und 17" so angeordnet sind, dass möglichst viel Objektreaktionsstrahlung ohne Reflexionen an den Wänden des Gehäuses 2 durch Filterkombinationen 18' und 18" von den Photoempfängern 17', 17" erfasst wird. Die Photoempfänger 17', 17" und die Filterkombinationen 18', 18" können in Abhängigkeit von der konkreten Anwendung gleich oder auch unterschiedlich sein.

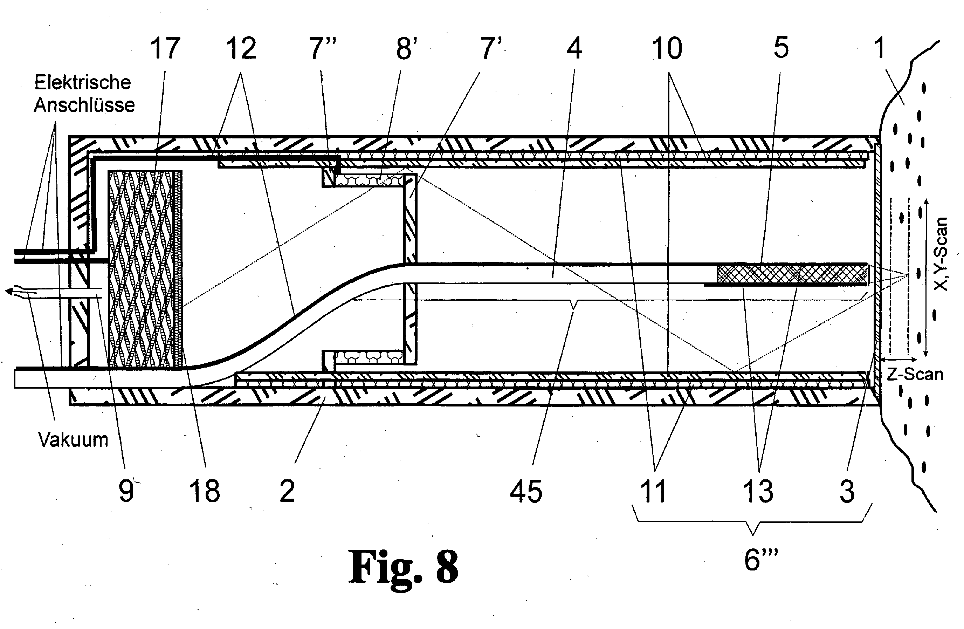

Für Photoempfänger 17, die keinen zentralen Durchlass haben, wie z.B. Sekundärelektronenvervielfacher (SEV bzw. PMT), ist eine alternative Ausführung in Fig. 8 dargestellt. In dieser Anordnung wird die Faser 4 in einem axial beweglichen Rahmen 7' befestigt, der eine Kreuzstegkonstruktion aufweist. Dadurch schattet der Rahmen 7' den Photoempfänger 17 nur minimal ab. Der Rahmen 7' ist auf einer ringförmigen bzw. axial segmentären Verstelleinheit 8' befestigt, der wiederum mit einem unbeweglichen ringförmigen bzw. segmentären Element 7" verbunden ist.

In allen Ausführungsvarianten gemäß den Figuren 6-8 kann auch eine Double-Clad-Large-Area-Core-PCF als Faser 4 eingesetzt werden, wenn ein Teil der Objektreaktionsstrahlung durch die Faser 4 zu dem externen Photoempfänger 36 geführt werden muss.

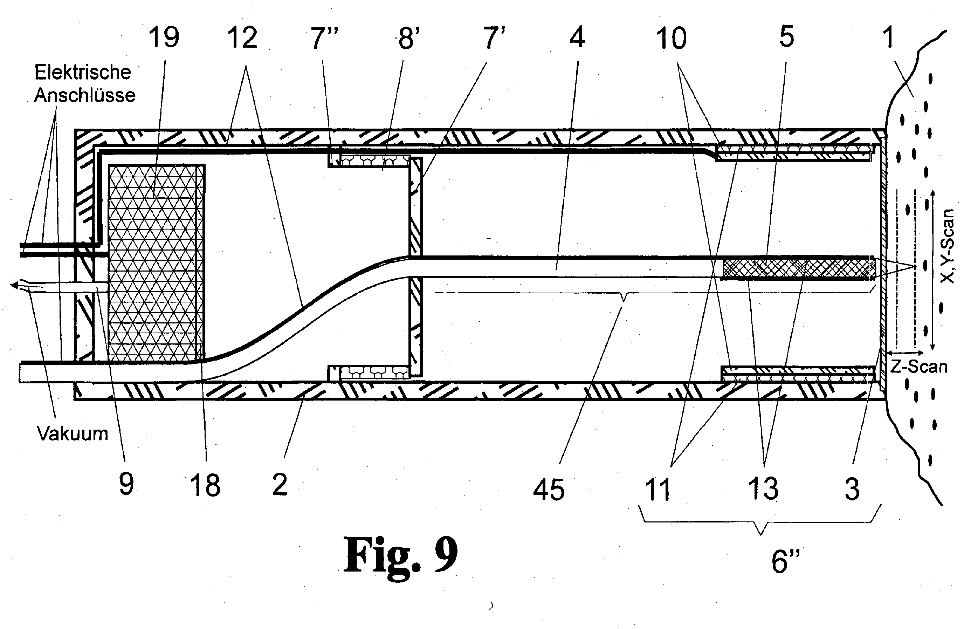

Fig.9 stellt eine Ausführung dar, bei der der Direkt-Scanner mit einem elektrostatischen Aktuator 6", gebildet aus Schichtelektroden 10, dielektrische Schicht 11, Zuleitung 12 und leitfähige Beschichtung 13, mit einem zweidimensionalen Bildsensor 19, z.B. einer CCD-Kamera inklusive zugehöriger Optik und Beleuchtung, kombiniert werden kann. Die Faser 4 wird in dieser Anordnung durch eine Double-Clad-Large-Area-Core-PCF gebildet, die sowohl für Abbildungs- als auch für mikrochirurgische oder Materialbearbeitungszwecke eingesetzt werden kann.

In allen Ausführungsbeispielen des Direkt-Scanners gemäß den Figuren 6-9 können für den elektrostatischen Aktuator 6" anstelle der leitfähigen Beschichtungen 10 und 13 zum Bewegen der Faser-Fokussieroptik-Einheit 45 auch ein Permanentmagnet 14 und Induktivelemente 15, wie in Fig. 4 dargestellt, oder am Gehäuse 2 und an der Faser-Fokussieroptik-Einheit 45 angebrachte Induktivelemente 15 und 16, wie in Fig. 5 gezeigt, verwendet werden.

### 2. Direkt-Scanner mit mehreren Faser-Fokussieroptik-Einheiten

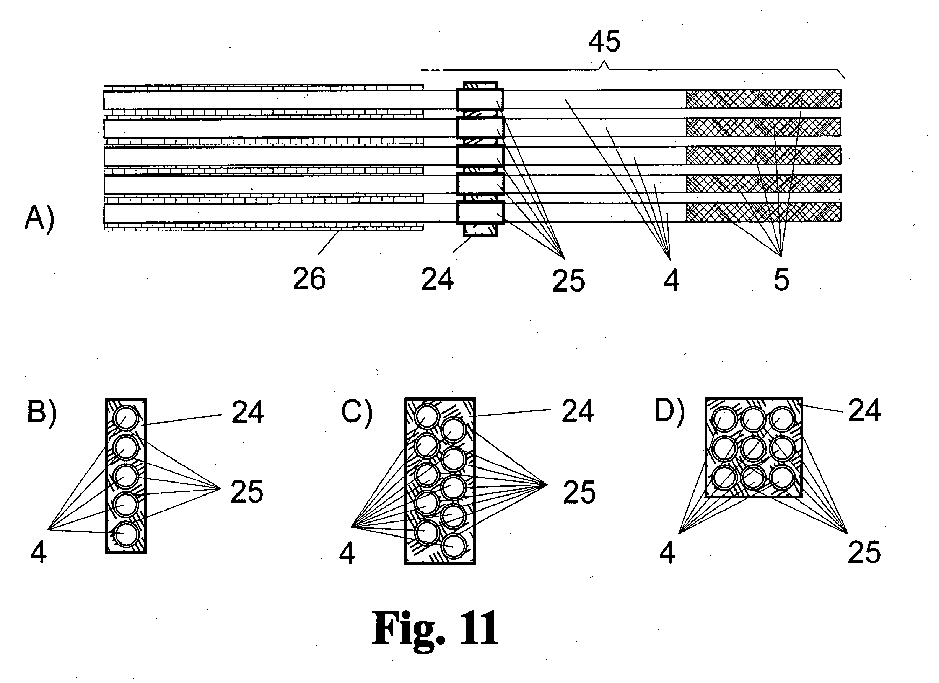

Der Direkt-Scanner kann auch als ein Mehrkanalgerät ausgeführt sein. In dieser Ausführung wird die Faser-Fokussieroptik-Einheit 45 durch eine Art mehradriges Kabel - wie in Fig. 11 gezeigt, ersetzt, wobei mindestens zwei Faser-Fokussieroptik-Einheiten 45 miteinander verbunden sind. Die Fasern 4 sind in eine flexible Bandage 26 eingebettet, die frei herausstehenden distalen Faserabschnitte sind vorzugsweise gleich lang und werden durch Versteifungselemente 25 in einer Halterung 24 fixiert. Die Versteifungselemente 25 dienen zur Einstellung von Schwingungseigenschaften der distalen Abschnitte der Faser-Fokussieroptik-Einheit 45. Sie können auch zur unmittelbaren Fixierung der einzelnen Miniatur-Fokussieroptiken 5 eingesetzt werden. Durch die Halterung 24 werden die Fasern 4 in einem geometrischen Muster anwendungsabhängig angeordnet, z.B. in einer Reihe (Fig. 11 B) oder zwei Reihen (Fig. 11 C) oder als Matrix (Fig. 11 D) symmetrisch oder asymmetrisch. Anwendungsabhängig sind auch noch anders gestaltete geometrische Muster möglich.

Die Fokussieroptiken 5 können abhängig vom Anwendungsfall in einer geraden (nach Fig. 2a) oder abgewinkelten (nach Fig. 2b) Variante verwendet werden.
Die Faserhalterung 24 wird entweder mit dem Scan-Aktuator 6 bzw. speziell einem Piezoaktuator 6' (gemäß Fig. 2a bzw. 2b) fest verbunden, oder in die axial beweglichen Scanner- oder Rahmenhalterung 7 bzw. 7' (Ausführungsvarianten nach Fig. 3-5, 9) integriert.
Die Art der eingesetzten Fasern 4 ist anwendungsabhängig, z.B. werden für MPI vorteilhaft Double-Clad-Large-Area-Core-PCF verwendet, für andere Anwendungen genügen Large-Area-Core-PCF.

### 2.1 Multi-Channel Scanning Imaging

In modifizierten Ausführung kann der Direkt-Scanner als Mehrkanal-Scanner für mehrkanalabgetastete Abbildung wie z.B. LSM, MPI oder OCT verwendet werden. Hier werden alle Faser-Fokussieroptik-Kombinationen 45 durch einen 2D-Scan-Aktuator 6 synchron über zugeordnete Teilbereiche bewegt. Aus den durch die einzelnen Fasern 4 weitergeleiteten Teilbildern wird das Gesamtbild zusammengesetzt. Dies wird durch eine einheitliche Brennweite aller einzelnen Miniatur-Fokussieroptiken 5 und eine gleichmäßige Leistungsverteilung der Anregungsstrahlung erreicht.

Durch das parallele Scannen mehrerer Faser-Fokussieroptik-Einheiten 45 wird die Bildaufnahmezeit wesentlich verkürzt - dies kann zur Eliminierung verschiedener Artefakte, verursacht durch Herzschlag, Atem oder eventuelles Zittern des Untersuchenden und/oder des Patienten, führen. Ebenso kann dadurch die Auflösung der Abbildung bei gleichbleibender Aufnahmezeit wesentlich erhöht oder der abgebildete Gesamtbereich vergrößert werden.
Eine eventuelle Tiefenverstellung erfolgt wie für Fig. 2 und 3 beschrieben.

### 2.2 Direkt-Multiphotonen-Tomographie

In einer weiteren Ausführung ist der Mehrkanal-Direkt-Scanner vergleichbar mit Beispiel 2.1 aufgebaut, jedoch sind die Brennweiten der Fokussieroptiken 5 von einzelnen Fasern 4 (oder Faser-Gruppen) unterschiedlich, wodurch gleichzeitig Abbildungen (Tomogramme) von mindestens zwei unterschiedlich tief im Objekt 1 liegenden Ebenen aufgenommen werden. Dadurch kann z.B. mit LSM oder MPI eine 3D-Abbildung eines Objekts 1 in einem Schritt aufgenommen werden. Des Weiteren kann die Aufnahmezeit von Tomogrammen in größeren Tiefenbereichen bzw. mit einer höheren Tiefenauflösung (mehrere Abbildungsebenen) durch Verwendung der Tiefenverstellung wesentlich verkürzt werden, wodurch wiederum die im Beispiel 2.1 genannten Artefakte eliminiert werden.

### 2.3 Direkt-Scanner unter Verwendung verschiedener Abbildungs- und/oder Messverfahren

In einer weiteren Ausführung ist der Mehrkanal-Direkt-Scanner wie in den Beispielen 2.1 oder 2.2 aufgebaut, wobei jedoch die einzelnen Fasern 4 (oder Faser-Gruppen) mit unterschiedlichen Fokussieroptiken 5 versehen sind. Diese werden für unterschiedliche optische Abbildungs- und/oder Messverfahren parallel verwendet, z.B. für die gleichzeitige bzw. schnelle sequenzielle MPI bzw. MP-Tomographie und OCT. In diesem Fall sind die Faser-Fokussieroptik-Einheiten 45 für das entsprechende Abbildungs- oder Messverfahren optimiert und ihre schwingungstechnischen Eigenschaften aneinander angeglichen.
Der Mehrkanal-Direkt-Scanner kann durch diese optimierten, angeglichenen Eigenschaften auch zur gleichzeitigen Messung verschiedener Charakteristika der Objektreaktionsstrahlung, z.B. Fluoreszenz-Polarisation, Kohärenz oder Objektabbildung, eingesetzt werden.

### 2.4 Mehrkanal-Direkt-Scanner mit Vereinigung von Abbildungs- und Bearbeitungsfunktion

In einer multifunktionalen Ausführung ist der Mehrkanal-Direkt-Scanner gemäß einem der Beispiele 2.1 bis 2.3 aufgebaut, aber die Fokussieroptiken 5 von einzelnen Fasern 4 bzw. Faser-Gruppen und die Faser 4 sind unterschiedlich. Einige der Faser-Fokussieroptik-Kombinationen 4, 5 sind für Abbildungs- (z.B. mit LSM oder MPI) und die anderen für Mikrochirurgie- oder Materialbearbeitungszwecke (z.B. mit Multiphotonen-Laserablation) optimiert, dadurch können Abbildung und Verarbeitung parallel oder schnell abwechselnd durch unterschiedliche Faser-Fokussieroptik-Einheiten 45 oder ganze Gruppen davon erfolgen.

### 2.5 Vereinigung mehrerer Einzelfaser-Direkt-Scanner

Durch Zusammenfügung von vorzugsweise gleichen Varianten des Direkt-Scanners gemäß den Figuren 2 bis 9, indem die Gehäuse 2 der Einzelfaser-Scanner starr miteinander verbunden werden (nicht separat gezeichnet), wird entweder die hochaufgelöst abtastbare Fläche des Objekts 1 vergrößert, z.B. paralleles MPI, oder ein Mehrkanalgerät geschaffen, z.B. OCT-Mehrkanal-Direkt-Scanner.

Die oben beschriebenen Anordnungen werden vorzugsweise genutzt, um Operation im mittleren und hinteren Augenabschnitt vorzunehmen, insbesondere zur Bearbeitung der Linse derart, dass durch präzise Schnitte die Elastizität verbessert werden kann, die Retina präzise bearbeitet, Gefäße bei der altersbedingten Makula-Degeneration verschlossen bzw. entfernt werden können, unerwünschte Zellen entfernt bzw. inaktiviert werden, Kanäle zur Druckentlastung gebohrt werden können und komplizierte Eingriffe um den Sehnerv durchgeführt werden können.
Anordnungen nach den Ausführungsbeispielen 1-4 können ferner genutzt werden, um präzise Operationen im Bereich des Mittelohrs durchzuführen.
Des Weiteren kommen die erfindungsgemäßen Ausführungen 1-4 für Operationen im Hirnbereich mit hoher Präzision und minimalem invasiven Eingriff zum Einsatz, um beispielsweise Tumorzellen optisch zu inaktivieren ohne benachbarte Hirnregionen zu schädigen.
Die Ausführungen 1-4 können außerdem vorteilhaft genutzt werden, um Operation im Bereich der Wirbelsäule durchzuführen, ohne benachbarte Nerven zu schädigen.
Die Anordnungen in den Ausführungsbeispielen 1-4 werden zweckmäßig auch dazu genutzt, um in gezüchteten Hautprodukten (tissue-engineered products?) unerwünschte Zellen und Mikroorganismen zu inaktivieren bzw. zu entfernen oder um Operationen innerhalb eines Embryonen mit hoher Präzision durchzuführen

Besonders geeignet sind Anordnungen gemäß den obigen Beispielen 1-4, um Mikrobearbeitungen im wässrigen Milieu vorzunehmen, insbesondere um optische Transfektionen für einen Molekültransfer (z.B. von DNS) im Körperinneren zu realisieren oder um injizierte Stammzellen, die sich unerwünscht differenzieren, optisch zu inaktivieren.

### Bezugszeichenliste

- 1: Objekt

- 2: Gehäuse
- 2': Gehäusedeckel
- 2": flexibler Schlauch
- 3: Fenster
- 4: (optische) Faser
- 45: Faser-Fokussier-Optikeinheit
- 5: (Miniatur-) Fokussieroptik
- 5': (refraktives) Kugellinsensegment
- 5": erste (objektseitige) GRIN-Linse
- 5'": zweite GRIN-Linse
- 5*: Diffraktivoptik
- 5**: Umlenkelement
- 6: Scan-Aktuator
- 6': Piezo-Scanner
- 6": elektrostatischer Aktuator
- 6'": elektromagnetischer Aktuator
- 7: axial bewegliche Scannerhalterung
- 7': axial bewegliche Rahmenhalterung
- 7": unbewegliche Rahmenhalterung
- 8: axiale Verstelleinheit
- 8': ringförmige axiale Verstelleinheit
- 9: Vakuumleitung

- 10: Schichtelektroden
- 11: dielektrische Schicht
- 12: Leitung
- 13: leitfähige Beschichtung
- 14: Permanentmagnet
- 15, 16: Induktivelemente
- 17, 17', 17": Photoempfänger
- 18, 18', 18": Filterkombination
- 19: (flächenhafter) Bildsensor
- 20: zentraler Bereich
- 21: mikrostrukturierter Bereich
- 22: Mantel
- 23: äußere Beschichtung
- 24: Faserhalterung
- 25: Versteifungselemente
- 26: flexible Bandage

- 30: Bestrahlungs- und Detektionseinrichtung
- 31: Femtosekundenlaser
- 32: Shutter
- 33: Abschwächer
- 34: (dichroitischer) Teilerspiegel
- 35: Mikroskopoptik
- 36: Photonendetektor (PMT)
- 37: Computer (PC)

## Patentansprüche

1. Verfahren zur Erzeugung mikroskopisch hochaufgelöster Abbildungen in der Endoskopie auf Basis von lasergenerierter Objektreaktionsstrahlung, **gekennzeichnet durch** die Schritte:
- gepulstes Einstrahlen fokussierter Anregungsstrahlung von einem Lasersystem (31) in ein Objekt (1) mittels einer Transmissions-Fokussier-Optikeinheit (45), bestehend aus einem Transmissionssystem (4;35) und einer mit dessen Ende starr verbundenen Miniatur-Fokussieroptik (5) hoher numerischer Apertur größer 0,55, zur Auslösung einer lokalen Reaktionsstrahlung des Objekts (1) im Mikrometer- bis Nanometerbereich,
- mindestens zweidimensionale Scanbewegung des distalen Endes der Transmissions-Fokussier-Optikeinheit (45) mit der starr verbundenen Miniatur-Fokussieroptik (5) durch in x- und y-Richtung geordnetes Bewegen des distalen Endes mittels eines Scan-Aktuators (6) innnerhalb eines Gehäuses (2) über einem optischen Fenster (3) zum sukzessive örtlich veränderten Positionieren der Anregungsstrahlung gegenüber dem am Fenster (3) anliegenden Objekt (1) und örtlich zugeordneten hochaufgelösten Abtasten von Objektreaktionsstrahlung und
- Übertragung von örtlich geordnet fortschreitend abgetasteter Objektreaktionsstrahlung mittels der Transmissions-Fokussier-Optikeinheit (45) an ein bildgebendes System (30) mit Photonendetektor (36).

2. Verfahren nach Anspruch 1, **dadurch gekennzeichnet, dass** ein dritter zu der zweidimensionalen Scanbewegung orthogonaler Scanvorgang durch axiales Bewegen der Transmissions-Fokussier-Optikeinheit (45) erfolgt zur Veränderung der Tiefe einer aus dem zweidimensionalen Scanbewegung erzeugten Bildaufnahme der Objektreaktionsstrahlung in einer durch die Miniatur-Fokussieroptik (5) vorgegebenen Fokusebene.

3. Verfahren nach Anspruch 1 oder 2, **dadurch gekennzeichnet, dass** die Miniatur-Fokussieroptik (5) und eine zu der Bestrahlungs- und Detektionseinrichtung (30) gehörige Optik (35), die eine direkte optische Kopplung zu der Bestrahlungs- und Detektionseinrichtung (30) herstellt, mittels einer starren Verbindung als Transmissions-Fokussier-Optikeinheit (45) gleichzeitig scannend bewegt werden, wobei in axialer Richtung eine Relativbewegung zur Einstellung der Tiefe der Fokusebene im Objekt (1) vorgenommen werden kann.

4. Verfahren nach Anspruch 1 oder 2, **dadurch gekennzeichnet, dass** die Miniatur-Fokussieroptik (5) und eine optische Faser (4), die eine optische Kopplung zu der Bestrahlungs- und Detektionseinrichtung (30) herstellt, mittels einer starren Verbindung als Transmissions-Fokussier-Optikeinheit (45) gleichzeitig scannend bewegt werden.

5. Verfahren nach Anspruch 4, **dadurch gekennzeichnet, dass** durch eine Art Faserbündel aus mehreren Faser-Fokussier-Optikeinheiten (45) simultan eine mehrkanalige Abtastung des Objekts (1) durchgeführt wird.

6. Verfahren nach Anspruch 2, **dadurch gekennzeichnet, dass** vom Objekt (1) emittierte Strahlung mittels zeitkorrelierter Einzelphotonen-Zählung detektiert wird.

7. Miniaturisierter mikroskopischer Kopf für endoskopische Anwendungen, bei dem eine Transmissionsoptik zur Zufuhrung einer Anregungsstrahlung, eine Fokussieroptik zum gebündelten Energieeintrag der Anregungsstrahlung in ein Objekt (1), und eine Scaneinrichtung zum Ändern des Ortes des Energieeintrags in einem Gehäuser (2) angeordnet sind, wobei die Fokussieroptik durch ein Fenster (3) im Gehäuse (2) die Wirkungsstrahlung in das Objekt (1) fokussiert, **dadurch gekennzeichnet, dass**
- die Fokussieroptik eine Miniatur-Fokussieroptik (5) ist, die einen Durchmesser von weniger als 2 mm und eine numerische Apertur von NA > 0,55 aufweist, wobei die Anregungsstrahlung mittels der Miniatur-Fokussieroptik (5) lokal auf einen Bereich von weniger als 100 µm begrenzt in das Objekt (1) fokussiert ist,
- die Transmissionsoptik (4) mit ihrem im Gehäuse (2) eines typischen endoskopischen Handteils befindlichen Ende starr mit der Miniatur-Fokussieroptik (5) verbunden ist und eine Transmissions-Fokussier-Optikeinheit (45) bildet, und
- die Scaneinrichtung mindestens einen in einer Ebene zweidimensional beweglichen Scan-Aktuator (6) zum in x- und y-Richtung geordneten Bewegen der Miniatur-Fokussieroptik (5) zum örtlich veränderten Positionieren der Wirkungsstrahlung gegenüber dem Fenster (3) des Gehäuses (2) aufweist.

8. Anordnung nach Anspruch 7, **dadurch gekennzeichnet, dass** die Scaneinrichtung weiterhin eine axial bewegliche Verstelleinheit (8) zur axialen Bewegung der Transmissions-Fokussier-Optikeinheit (45) aufweist, wobei die Transmissions-Fokussier-Optikeinheit (45) inh der Verstelleinheit (8) axial fest eingespannt ist.

9. Anordnung nach Anspruch 7, **dadurch gekennzeichnet dass** die Transmissions-Fokussier-Optikeinhet (45) aus der Miniatur-Fokussieroptik (5) und einer optischen Faser (4) starr zusammengesetzt ist, wobei die Transmissions-fokussier-Optikeinheit (45), in der Verstelleinheit (8) axial fest eingespannt ist.

10. Anordnung nach Anspruch 7, **dadurch gekennzeichnet, dass** die Transmissions-Fokussier-Optikeinheit (45) aus der Miniatur-Fokussieroptik (5) und einem verspiegelten Hohlleiter starr zusammengesetzt ist, wobei die Transmission-Fokussier-Optikeinheit (45) in der Verstelleinheit (8) axial fest eingespannt ist

11. Anordnung nach Anspruch 7, **dadurch gekennzeichnet, dass** die Transmission-Fokussier-Optikeinheit (45) aus der Miniatur-Fokussieroptik (5) und einer Mikroskopoptik (35) einer Bestrahlungs- und Detektionseinrichtung (30) besteht wobei der zweidimensionale Scan-Aktuator (6) und die axiale Verstelleinheit (8) ausschließlich an die Miniatur-Fokussieroptik (5) gekoppelt sind,

12. Anordnung nach Anspruch 7, **dadurch gekennzeichnet, dass** die Fokussleroptik (5) eine GRIN-Optik mit gerundeter Endfläche ist.

13. Anordnung nach Anspruch 7, **dadurch gekennzeichnet, dass** die Fokussieroptik (5) eine GRIN-Optik mit vorgeordnetem refraktivem Kugellinsenelement (5') ist.

14. Anordnung nach Anspruch 7, **dadurch gekennzeichnet, dass** die Fokussieroptik (5) eine zweilinsige GRIN-Optik (5",5"') und eine diffraktive Optik (5') enthält.

15. Anordnung nach Anspruch 7, **dadurch gekennzeichnet, dass** die axiale Verstelleinheit (8) starr mit dem zweidimensionalen Scan-Aktuator (6) verbunden und der Scan-Aktuator (6, 6',6",6"') in einer axial beweglichen Scannerhalterung (7) befestigt ist.

16. Anordnung nach Anspruch 9, **dadurch gekennzeichnet, dass** die Transmissionsoptik eine mikrostrukturierte Faser (4) vom Typ Double-Clad-Large-Area-Core-Photonic-Crystal-Fiber ist, die Femtosekunden-Laserstrahlung nahezu dispersionsfrei überträgt.

17. Anordnung nach Anspruch 9, **dadurch gekennzeichnet, dass** die Transmissionsoptik eine Faser (4) vom Typ Large-Area-Core-Photonic-Crystal-Fiber ist.

18. Anordnung nach Anspruch 9, **dadurch gekennzeichnet, dass** im Gehäuse (2) um die Faser (4) herum mindestens ein Photoempfänger (17) zum direkten Aufnehmen der Objektreaktionsstrahlung angeordnet ist.

19. Anordnung nach Anspruch 9, **dadurch gekennzeichnet, dass** im Gehäuse (2) um die Faser (4) herum mehrere Photoempfänger (17) zum direkten Aufnehmen unterschiedlicher spektraler Bestandteile der Objektreaktionsstrahlung angeordnet sind.

20. Anordnung nach Anspruch 9, **dadurch gekennzeichnet, dass** im Gehäuse (2) mehrere Fasern (4) parallel angeordnet und die Fasern (4) in einer Faserhalterung (24) parallel geführt sind.

21. Anordnung nach Anspruch 20, **dadurch gekennzeichnet dass** die Fasern (4) in einer flexiblen Bandage (26) eingebettet sind und Versteifungselemente (25) zur Schwingungsunterdrückung aufweisen.

22. Anordnung nach Anspruch 7, **dadurch gekennzeichnet dass** das Gehäuse (2) rohrförmig ausgebildet und an dessen Stirnseiten durch einen Deckel (2') und das Fenster (3) hermetisch abgedichtet ist.

23. Anordnung nach Anspruch 7, **dadurch gekennzeichnet, dass** das Gehäuse (2) mindestens eine ebene Seitenwand aufweist, wobei in der ebenen Seitenwand des Gehäuses (2) seitlich ein Fenster (3) angebracht und das Gehäuse (2) mit stirnseitigen Deckeln (2') hermetisch abgedichtet ist.

24. Anordnung nach Anspruch 23, **dadurch gekennzeichnet, dass** die Miniatur-Fokussieroptik (5) ein strahlungsumlenkendes Element (5**) aufweist, so dass die Anregungsstrahlung lateral aus der Transmissions-Fokussier-Optikeinheit (45) austritt und durch das seitlich angeordnete Fenster (3) auf das Objekt (1) fokussierbar ist, wobei die Verstelleinheit (8) für eine erste laterale Scan-Dimension am Objekt (1) und der Scan-Aktuator (6) für eine zweite laterale Scan-Dimension sowie den Tiefenscan im Objekt (1) vorgesehen sind.

25. Anordnung nach Anspruch 22 oder 23, **dadurch gekennzeichnet, dass** das hermetisch abgedichtete Gehäuse (2) evakuiert ist.

## Claims

1. Method for generating high-resolution microscopic images in the endoscopy based on laser-induced object response radiation, **characterized by** the following steps:
- focusing pulsed excitation radiation from a laser system (31) into an object (1) by means of a transmission focusing optics unit (45) comprising a transmission system (4;35) and miniature focusing optics (5) having a high numerical aperture greater than 0.55 which are rigidly connected to the end of the transmission system (4;35) to trigger a local response radiation of the object (1) in the micrometer to nanometer range,
- executing a scanning movement of the distal end of the transmission focusing optics unit (45) with the rigidly connected miniature focusing optics (5) in at least two dimensions by moving the distal end in X and Y direction in an orderly manner by means of a scan actuator (6) within a housing (2) via an optical window (3) for the successive locally changed positioning of the excitation radiation relative to the object (1) contacting the window (3) and for locally allocated high-resolution scanning of object response radiation, and
- transmitting the object response radiation which is scanned in an orderly locally progressive manner by the transmission focusing optics unit (45) to an image-generating system (30) with a photon detector (36).

2. Method according to claim 1, **characterized in that** a third scanning process is carried out orthogonal to the two-dimensional scanning movement by axial movement of the transmission focusing optics unit (45) for changing the depth of an image recording of the object response radiation generated from the two-dimensional scanning movement into a focal plane predetermined by the miniature focusing optics (5).

3. Method according to claim 1 or 2, **characterized in that** the miniature focusing optics (5) and optics (35) which are associated with the illumination and detection device (30) and which produce a direct optical coupling with the illumination and detection device (30) are moved in a scanning motion simultaneously as a transmission focusing optics unit (45) by means of a rigid connection, wherein a relative movement can be carried out in axial direction for adjusting the depth of the focal plane in the object (1).

4. Method according to claim 1 or 2, **characterized in that** the miniature focusing optics (5) and an optical fiber (4) which produces an optical coupling with the illumination and detection device (30) are moved simultaneously in a scanning manner by means of a rigid connection as a transmission focusing optics unit (45).

5. Method according to claim 4, **characterized in that** a multichannel scanning of the object (1) is carried out simultaneously by a type of fiber bundle comprising a plurality of fiber focusing optics units (45).

6. Method according to claim 2, **characterized in that** the radiation emitted by the object (1) is detected by means of time-correlated single-photon counting.

7. Miniaturized microscopic head for endoscopic applications, comprising transmission optics for supplying an excitation radiation, focusing optics for introducing bundled excitation radiation into an object (1), and a scanning device for changing the location of the energy input, said transmission optics, said focusing optics, and said scanning device being arranged in a housing (2), wherein the focusing optics focus the active radiation into the object (1) through a window (3) in the housing (2), **characterized in that**
- said focusing optics are miniature focusing optics (5) having a diameter of less than 2 mm and a numerical aperture of NA > 0.55, wherein the excitation radiation is focused by the miniature focusing optics (5) so as to be limited locally on an area of less than 100 µm in the object (1),
- the end of said transmission optics (4) in the housing (2) of a typical endoscopic hand part is rigidly connected to said miniature focusing optics (5), wherein both form a transmission focusing optics unit (45), and
- said scanning device has at least one scan actuator (6) arranged to cause a two-dimensional movement in a plane for moving the miniature focusing optics (5) in X and Y direction in an orderly manner for the locally changed positioning of the active radiation relative to the window (3) of the housing (2).

8. Arrangement according to claim 7, **characterized in that** the scanning device further has an axially movable adjusting unit (8) for axial movement of the transmission focusing optics unit (45), wherein the transmission focusing optics unit (45) is clamped in the adjusting unit (8) so as to be fixed axially.

9. Arrangement according to claim 7, **characterized in that** the transmission focusing optics unit (45) is rigidly assembled from the miniature focusing optics (5) and an optical fiber (4), wherein the transmission focusing optics unit (45) is clamped in the adjusting unit (8) so as to be fixed axially.

10. Arrangement according to claim 7, **characterized in that** the transmission focusing optics unit (45) is rigidly assembled from the miniature focusing optics (5) and a reflective waveguide, wherein the transmission focusing optics unit (45) is clamped in the adjusting unit (8) so as to be fixed axially.

11. Arrangement according to claim 7, **characterized in that** the transmission focusing optics unit (45) comprises the miniature focusing optics (5) and microscope optics (35) of an illumination and detection device (30), wherein the two-dimensional scan actuator (6) and the axial adjusting unit (8) are connected exclusively to the miniature focusing optics (5).

12. Arrangement according to claim 7, **characterized in that** the focusing optics (5) are GRIN optics with a rounded end face.

13. Arrangement according to claim 7, **characterized in that** the focusing optics (5) are GRIN optics preceded by a refractive spherical lens element (5').

14. Arrangement according to claim 7, **characterized in that** the focusing optics (5) contain two-lens GRIN optics (5", 5"') and diffractive optics (5*).

15. Arrangement according to claim 7, **characterized in that** the axial adjusting unit (8) is rigidly connected to the two-dimensional scan actuator (6) and the scan actuator (6, 6', 6", 6"') is fastened in an axially movable scanner holder (7).

16. Arrangement according to claim 9, **characterized in that** the transmission optics comprise a microstructured fiber (4) of the double-clad large-area core photonic-crystal fiber type which transmits the femtosecond laser radiation virtually without dispersion.

17. Arrangement according to claim 9, **characterized in that** the transmission optics comprise a fiber (4) of the large-area core photonic-crystal fiber type.

18. Arrangement according to claim 9, **characterized in that** at least one photoreceiver (17) is arranged around the fiber (4) in the housing (2) for directly receiving the object response radiation.

19. Arrangement according to claim 9, **characterized in that** a plurality of photoreceivers (17) are arranged around the fiber (4) in the housing (2) for directly receiving different spectral components of the object response radiation.

20. Arrangement according to claim 9, **characterized in that** a plurality of fibers (4) are arranged in parallel and the fibers (4) are guided in parallel in a fiber holder (24) in the housing (2).

21. Arrangement according to claim 20, **characterized in that** the fibers (4) are embedded in a flexible band (26) and have stiffening elements (25) for suppressing oscillation.

22. Arrangement according to claim 7, **characterized in that** the housing (2) is tubular and is hermetically sealed at its end sides by a cover (2') and the window (3).

23. Arrangement according to claim 7, **characterized in that** the housing (2) has at least one plane side wall, wherein a window (3) is arranged laterally in the plane side wall of the housing (2) and the housing is hermetically sealed by end covers (2').

24. Arrangement according to claim 23, **characterized in that** the miniature focusing optics (5) have a radiation-deflecting element (5**) so that the excitation radiation exits laterally from the transmission focusing optics unit (45) and can be focused on the object (1) through the laterally arranged window (3), wherein the adjusting unit (8) is provided for a first lateral scan dimension at the object (1) and the scan actuator (6) is provided for a second lateral scan dimension and for the depth scan in the object (1).

25. Arrangement according to claim 22 or 23, **characterized in that** the hermetically sealed housing (2) is evacuated.

## Revendications

1. Procédé de génération d'images microscopiques à haute résolution en endoscopie sur la base de rayonnement de réponse d'objet induit par laser, **caractérisé par** les étapes suivantes:
- focaliser un rayonnement d'excitation pulsé d'un système laser (31) dans un objet (1) au moyen d'une unité optique de transmission et de focalisation (45) comprenant un système de transmission (4;35) et une optique de focalisation miniature (5) ayant une ouverture numérique grande, plus grande que 0,55, qui est reliée rigidement à l'extrémité distale du système de transmission (4;35) pour déclencher un rayonnement de réponse local de l'objet (1) de l'ordre du micromètre et du nanomètre,
- effectuer un mouvement de balayage, à au moins deux dimensions, de l'extrémité distale de l'unité optique de transmission et de focalisation (45) avec l'optique de focalisation miniature (5) étant reliée rigidement en déplaçant l'extrémité distale dans la direction X et Y d'une manière ordonnée au moyen d'un actuateur de balayage (6) dans un boîtier (2) via une fenêtre optique (3) pour positionner le rayonnement d'excitation d'une manière successive et localement modifiée relatif à l'objet (1) qui est en contact avec la fenêtre (3) et pour balayer le rayonnement de réponse d'objet à haute résolution et de manière attribuée localement, et
- transmettre le rayonnement de réponse d'objet qui est balayé de manière localement progressive et ordonnée au moyen de l'unité optique de transmission et de focalisation (45) à un système de génération d'images (30) avec un détecteur de photons (36).

2. Procédé selon la revendication 1, **caractérisé en ce qu'**un troisième processus de balayage est effectué orthogonale par rapport au mouvement de balayage à deux dimensions par un mouvement axial de l'unité optique de transmission et de focalisation (45) pour modifier la profondeur d'un enregistrement d'images du rayonnement de réponse d'objet généré du mouvement de balayage à deux dimensions dans un plan focal prédéterminé par l'optique de focalisation miniature (5).

3. Procédé selon la revendication 1 ou 2, **caractérisé en ce que** l'optique de focalisation miniature (5) et une optique (35), qui est associée au dispositif d'illumination et de détection (30) et qui produit un couplage optique direct au dispositif d'illumination et de détection (30), sont déplacées en balayant simultanément comme unité optique de transmission et de focalisation (45) au moyen d'une liaison rigide, un mouvement relatif pouvant être effectué en direction axiale pour régler le profondeur du plan focal dans l'objet (1).

4. Procédé selon la revendication 1 ou 2, **caractérisé en ce que** l'optique de focalisation miniature (5) et une fibre optique (4), qui produit un couplage optique avec le dispositif d'illumination et de détection (30), sont déplacées en balayant simultanément comme unité optique de transmission et de focalisation (45) au moyen d'une liaison rigide.

5. Procédé selon la revendication 4, **caractérisé en ce qu'**un balayage à plusieurs canaux de l'objet (1) est effectué simultanément par une sorte de faisceau de fibre comprenant une pluralité d'unités optiques de focalisation de fibre (45).

6. Procédé selon la revendication 2, **caractérisé en ce que** le rayonnement émis par l'objet (1) est détecté au moyen de comptage de photons uniques corrélé en temps.

7. Tête microscopique miniaturisée pour des applications endoscopiques, comprenant une optique de transmission pour fournir un rayonnement d'excitation, une optique de focalisation pour introduire un rayonnement d'excitation rassemblé dans un objet (1), et un dispositif de balayage pour modifier le lieu de l'apport d'énergie, ladite optique de transmission, ladite optique de focalisation et ledit dispositif de balayage étant disposés dans un boîtier (2), l'optique de focalisation focalisant le rayonnement actif dans l'objet (1) via une fenêtre (3) dans le boîtier (2), **caractérisée en ce que**
- ladite optique de focalisation est une optique de focalisation miniature (5) ayant un diamètre de moins de 2 mm et une ouverture numérique de NA > 0,55, le rayonnement d'excitation étant focalisé par l'optique de focalisation miniature (5) de manière à être limité localement sur une zone de moins de 100 µm dans l'objet (1),
- l'extrémité de ladite optique de transmission (4) dans le boîtier (2) d'une pièce à main endoscopique typique est reliée rigidement à ladite optique de focalisation miniature (5), les deux formant une unité optique de transmission et de focalisation (45), et
- ledit dispositif de balayage a au moins un actuateur de balayage (6) disposé pour causer un mouvement à deux dimensions dans un plan pour déplacer l'optique de focalisation miniature (5) dans la direction X et Y d'une manière ordonnée pour positionner le rayonnement actif d'une manière localement modifiée relatif à la fenêtre (3) du boîtier (2).

8. Agencement selon la revendication 7, **caractérisé en ce que** le dispositif de balayage a de plus une unité de réglage (8) qui est déplaçable axialement pour le mouvement axial de l'unité optique de transmission et de focalisation (45), l'unité optique de transmission et de focalisation (45) étant serrée axialement dans l'unité de réglage (8) de manière fixe.

9. Agencement selon la revendication 7, **caractérisé en ce que** l'unité optique de transmission et de focalisation (45) est assemblée rigidement de l'optique de focalisation miniature (5) et d'une fibre optique (4), l'unité optique de transmission et de focalisation (45) étant serrée axialement dans l'unité de réglage (8) de manière fixe.

10. Agencement selon la revendication 7, **caractérisé en ce que** l'unité optique de transmission et de focalisation (45) est assemblée rigidement de l'optique de focalisation miniature (5) et d'un guide d'ondes réflectif, l'unité optique de transmission et de focalisation (45) étant serrée axialement dans l'unité de réglage (8) de manière fixe.

11. Agencement selon la revendication 7, **caractérisé en ce que** l'unité optique de transmission et de focalisation (45) comprend l'optique de focalisation miniature (5) et une optique de microscope (35) d'un dispositif d'illumination et de détection (30), l'actuateur de balayage (6) à deux dimensions et l'unité de réglage axiale (8) étant reliés exclusivement à l'optique de focalisation miniature (5).

12. Agencement selon la revendication 7, **caractérisé en ce que** l'optique de focalisation (5) est une optique GRIN avec une surface d'extrémité arrondie.

13. Agencement selon la revendication 7, **caractérisé en ce que** l'optique de focalisation (5) est une optique GRIN précédée d'un élément de lentille sphérique réfractif (5').

14. Agencement selon la revendication 7, **caractérisé en ce que** l'optique de focalisation (5) contient une optique GRIN à deux lentilles (5", 5"') et une optique diffractive (5*).

15. Agencement selon la revendication 7, **caractérisé en ce que** l'unité de réglage axiale (8) est reliée rigidement à l'actuateur de balayage (6) à deux dimensions et l'actuateur de balayage (6, 6', 6", 6"') est fixé dans un support de scanner (7) qui est déplaçable axialement.

16. Agencement selon la revendication 9, **caractérisé en ce que** l'optique de transmission comprend une fibre microstructurée (4) du type double-clad-large-area-core-photonic-crystal-fiber qui transmet le rayonnement laser à femtosecondes presque sans dispersion.

17. Agencement selon la revendication 9, **caractérisé en ce que** l'optique de transmission comprend une fibre (4) du type large-area-core-photonic-crystal-fiber.

18. Agencement selon la revendication 9, **caractérisé en ce qu'**au moins un photorécepteur (17) est disposé autour de la fibre (4) dans le boîtier (2) pour la réception directe du rayonnement de réponse d'objet.

19. Agencement selon la revendication 9, **caractérisé en ce qu'**une pluralité de photorécepteurs (17) sont disposés autour de la fibre (4) dans le boîtier (2) pour la réception directe de différents composants spectraux du rayonnement de réponse d'objet.

20. Agencement selon la revendication 9, **caractérisé en ce qu'**une pluralité de fibres (4) sont disposées en parallèle et les fibres (4) sont guidées dans un support de fibres (24) dans le boîtier (2).

21. Agencement selon la revendication 20, **caractérisé en ce que** les fibres (4) sont encastrées dans une bande flexible (26) et ont des éléments de raidissement (25) pour supprimer de l'oscillation.

22. Agencement selon la revendication 7, **caractérisé en ce que** le boîtier (2) est tubulaire et hermétiquement fermé à ses faces frontales par un couvercle (2') et la fenêtre (3).

23. Agencement selon la revendication 7, **caractérisé en ce que** le boîtier (2) a au moins une paroi latérale plane, une fenêtre (3) étant disposée latéralement dans la paroi latérale plane du boîtier (2) et le boîtier (2) est hermétiquement fermé par des couvercles frontaux (2').

24. Agencement selon la revendication 23, **caractérisé en ce que** l'optique de focalisation miniature (5) a un élément déviant le rayonnement (5**) de façon que le rayonnement d'excitation sort latéralement de l'unité optique de transmission et de focalisation (45) et peut être focalisé sur l'objet (1) via la fenêtre (3) qui est disposée latéralement, l'unité de réglage (8) étant fournie pour une première dimension de balayage latérale à l'objet (1) et l'actuateur de balayage (6) pour une deuxième dimension de balayage latérale et pour un balayage de profondeur dans l'objet (1).

25. Agencement selon la revendication 22 ou 23, **caractérisé en ce que** le boîtier (2) hermétiquement fermé est évacué.
